# EUROPEAN PATENT SPECIFICATION

(11) **EP 4 553 161 B1**
(45) Date of publication and mention of the grant of the patent: **13.05.2026**
(21) Application number: 24833794.1
(22) Date of filing: 10.09.2024
(51) Int. Cl.: C12N 15/64, C12N 15/70

(54) **ANTIBIOTIC-FREE PLASMID PRODUCTION SYSTEM AND USE THEREOF**
RESISTENZFREIES PLASMIDPRODUKTIONSSYSTEM UND DESSEN VERWENDUNG
SYSTÈME DE PRODUCTION DE PLASMIDES SANS ANTIBIOTIQUES ET SON UTILISATION

(30) Priority: 11.09.2023 CN 202311168432
(43) Date of publication of application: 14.05.2025
(73) Proprietor: Yunzhou Biosciences (Guangzhou) Co., Ltd., Guangzhou, Guangdong 510000 (CN)
(72) Inventor: SHI, Jinxiu, Guangzhou, Guangdong 510000 (CN); LIN, Yingjun, Guangzhou, Guangdong 510000 (CN); LAHN, Bruce, Guangzhou, Guangdong 510000 (CN)
(74) Representative: Patentanwälte Magenbauer & Kollegen Partnerschaft mbB
(86) International application number: PCT/CN2024/117856
(87) International publication number: WO 2025/055871

(56) References cited:
- WO-A1-2012/069551
- WO-A2-2006/029985
- CN-A- 106 715 681
- CN-A- 112 961 876
- CN-A- 117 187 281
- CN-A- 118 374 547
- US-A1- 2011 207 173
- US-A1- 2011 217 282
- US-A1- 2013 337 013
- US-A1- 2014 004 602
- SZPIRER C�DRIC Y ET AL: "Separate-component-stabilization system for protein and DNA production without the use of antibiotics", BIOTECHNIQUES, INFORMA HEALTHCARE, US, vol. 38, no. 5, 1 May 2005 (2005-05-01), pages 775 - 781, XP001538926, ISSN: 0736-6205, DOI: 10.2144/05385RR02
- CHEN ZHE, YAO JIANYUN, ZHANG PINGJING, WANG PENGXIA, NI SONGWEI, LIU TAO, ZHAO YI, TANG KAIHAO, SUN YAN, QIAN QIJUN, WANG XIAOXUE: "Minimized antibiotic-free plasmid vector for gene therapy utilizing a new toxin-antitoxin system", METABOLIC ENGINEERING, vol. 79, 1 September 2023 (2023-09-01), AMSTERDAM, NL, pages 86 - 96, XP093194256, ISSN: 1096-7176, DOI: 10.1016/j.ymben.2023.07.003
- WANG XIAONAN, CABALES AVANIEK, LI ZHENGHONG, ZHANG HAORAN: "Biosensor-assisted high performing cell selection using an E. coli toxin/antitoxin system", BIOCHEMICAL ENGINEERING JOURNAL, vol. 144, 1 April 2019 (2019-04-01), NL , pages 110 - 118, XP093291830, ISSN: 1369-703X, DOI: 10.1016/j.bej.2019.01.016
- WANG XIAOXUE; YAO JIANYUN; SUN YI-CHENG; WOOD THOMAS K.: "Type VII Toxin/Antitoxin Classification System for Antitoxins that Enzymatically Neutralize Toxins", TRENDS IN MICROBIOLOGY, vol. 29, no. 5, 17 December 2020 (2020-12-17), GB , pages 388 - 393, XP086534303, ISSN: 0966-842X, DOI: 10.1016/j.tim.2020.12.001

## Description

### Technical Field

The invention relates to the field of biotechnology, and in particular to a resistance-free plasmid production system and the use thereof.

### Background

In recent years, gene therapy methods have attracted more and more attention, especially DNA or RNA vaccines, which have overcome the defects of traditional vaccines or viral vector vaccines in many aspects. Whether it is a DNA or RNA vaccine or a viral vector vaccine, the demand for plasmids is usually very large.

Traditional plasmid systems have many defects in the application of gene therapy. On the one hand, traditional plasmids carry antibiotic marker genes, but the overexpression of resistance genes will have certain side effects on the metabolism and growth of *Escherichia coli,* and the spread of antibiotics in the environment will cause widespread antibiotic resistance in the population. At the same time, the residual antibiotics in the plasmids may also cause allergic reactions in sensitive individuals. On the other hand, traditional plasmids carry prokaryotic bacterial backbones, most of which contain unmethylated CpG sequences, which are easy to induce the innate immune response (CpG oligodeoxynucleotide and products of genes on the prokaryotic backbone of the plasmid can be immunogens). Whether antibiotic marker genes or prokaryotic bacterial backbones are carried, the sequences outside the exogenous gene expression cassette will account for too large a proportion of the plasmid, increasing the size of the plasmid, which not only affects the yield of the plasmid, but also leads to a decrease in transfection efficiency, which is a negative impact on gene therapy. Smaller supercoiled plasmids can reach cell nucleus more effectively and enable the exogenous gene to be expressed continuously and persistently. Therefore, plasmids that lack resistance genes and have shorter prokaryotic backbones have greater advantages.

In order to improve the safety of gene therapy, a new generation of plasmid backbone systems without antibiotic resistance markers have been developed, mainly including the following: 1. Complementation of malnutrition strains: The strain is modified by introducing a deletion or mutation into a gene essential for maintaining bacterial growth, resulting in malnutrition of the strain and inability to grow; then a plasmid carrying the deleted gene is introduced into the strain to restore the growth of the strain. 2. Toxicity-antitoxicity system: A toxic gene is inserted into the bacterial genome to make the bacteria unable to grow, and an anti-toxic gene is cloned into the plasmid and introduced into the strain to restore the growth of the strain. 3. Operator/repressor system: A certain repressor protein is introduced upstream of a growth-essential gene in the bacterial genome to inhibit the expression of the essential gene, and a plasmid comprising one or more operator gene sequences is introduced into the bacteria to competitively titrate the repressor, thereby allowing the expression of the gene. 4. Overexpression of growth-essential genes: Overexpression of certain growth-essential genes (fabl or murA) in *Escherichia coli* can reduce the sensitivity to antibacterial compounds thereof, but inhibitors are present in the culture medium and must be removed from the purified plasmid DNA. Although the above-mentioned novel resistance-free plasmid systems have solved the problem of using antibiotic marker genes, there are still two major problems: 1. The sequence outside the target gene expression cassette accounts for too large a proportion in the plasmid, resulting in an increase of plasmid length. Therefore, under the same unit weight, part of the plasmid yield is contributed by the ineffective prokaryotic sequence backbone, and the copy number of the target gene expression cassettes accounts for a relatively reduced proportion, affecting the yield of the target expression cassettes. 2. Although some systems do not require the use of antibiotics, other auxiliary inhibitors still need to be added, and these inhibitors cannot be guaranteed to be 100% removed in the final plasmid product, which will not only increase the cost of plasmid preparation, but also the impact of the remain thereof in gene therapy is uncontrollable. Therefore, these resistance-free vector systems cannot be effectively popularized at present.

Therefore, there remains a need in the art to develop a plasmid production system that does not require the use of antibiotics and other auxiliary inhibitors.

Chen Zhe et. al., Minimized antibiotic-free plasmid vector for gene therapy utilizing a new toxin-antitoxin system", Metabolic Engineering, vol. 79, 1 September 2023 (2023-09-01), pages 86-96, XP093194256,AMSTERDAM, NL) discloses an antibiotic-free plasmid vector with a minimized backbone utilizing a toxin-antitoxin (TA) system in which the Rs_0636/Rs_0637 TA pair was derived from the coral-associated bacterium *Rose-ivirga* sp. The toxin gene is integrated into the chromosome of *Escherichia coli* host cells, and a recombinant mammalian expression plasmid is constructed by replacing the antibiotic resistance gene with the antitoxin gene Rs_0637 (here named Tiniplasmid).

### Summary of the invention

In order to solve the defects of the prior art, the present invention provides a resistance-free plasmid production system and the use thereof. The production system does not require the use of antibiotics and other auxiliary inhibitors, and the copy number of some plasmids can reach 4-5 times when compared with the conventional plasmid, which improves the application safety and greatly reduces the production cost.

In order to achieve the above-mentioned object of the invention, the present invention provides the following technical solutions:
In one aspect, the present invention provides a plasmid production system, characterized in that it comprises host cell expression cassettes and a plasmid;
The host cell expression cassette comprises a toxic gene expression cassette and an anti-toxic gene expression cassette;
The toxic gene expression cassette comprises a promoter and a nucleic acid fragment 1 for transcribing a toxic gene product; the toxic gene product may comprise a gene and/or a non-coding RNA that is toxic to the host;
The anti-toxic gene expression cassette comprises a promoter, an operator and a nucleic acid fragment 2 for transcribing an anti-toxic gene product; the anti-toxic gene product has a resistance effect on the toxicity produced by the toxic gene product; wherein the anti-toxic gene product may comprise an anti-toxic gene gene product and/or a non-coding RNA that has a resistance effect on the toxicity of the toxic gene product;
The plasmid comprises a nucleic acid fragment 3 encoding a small RNA and an Ori replication origin, wherein the nucleic acid fragment 3 is reversely complementary to the nucleic acid fragment 1, thereby inhibiting the function of the toxic gene product.

In the plasmid production system of the present invention, the operon regulates the opening and closing of the promoter, thereby manipulating the expression of the anti-toxic gene. When the toxic gene product is expressed alone, the host cell cannot grow or its growth is inhibited; the product of the anti-toxic gene product has a resistance effect on the toxicity produced by the toxic gene product. When the toxic gene product and the anti-toxic gene product are expressed at the same time, the toxic effect of the toxic gene product is inhibited, and the host cell can grow normally.

In some embodiments, the operon is any one of arabinose operon, Lac operon, rhamnose catabolism operon, tryptophan operon, gab operon and Gal operon. The promoter regulated by the operon is any one of araBAD promoter, T7lac promoter, Tac promoter, Trc promoter, LacUV5 promoter, tetA promoter, rhaBAD, gabP, and galP1 promoter; the repressor gene of the operon is AraC, LacI, CAP, rhaS/R, tetracycline, csiR or galR, including but not limited to these.

In some embodiments, the nucleic acid fragment 3 is completely complementary to the nucleic acid fragment 1 reversely or has at least 20% reverse complementarity in the sequence or at least 10 consecutive bp reverse complementarity.

In some embodiments, the nucleic acid fragment 1 comprises a UTR sequence and a toxic gene, and the UTR sequence is selected from any one of 1) to 4):
1) the nucleic acid sequence shown in positions 1 to 252 of SEQ ID NO: 1;
2) a sequence in which one or more nucleotides are substituted, deleted or added in the sequence shown in 1) and has the same or similar function;
3) a sequence complementary to the sequence shown in 1) or 2);
4) a sequence that has at least 50% homology with the sequence shown in 1), 2) or 3) and has unchanged function.

In some embodiments, the UTR sequence can also be selected from the nucleic acid sequence shown in positions 1 to 64 of SEQ ID NO:2.

In some embodiments, the UTR sequence may also be selected from SEQ ID NO: 10, 12 and 14.

In the present invention, the toxic gene can be selected from any one of ccdB, ParE, MazF, Kid, HicA, RelE, VapC, Doc, RatA, HipA, Zeta, ToxN, YeeV, CptA, GhoT, Hok, TisB, SymE, and PasA; the anti-toxic gene product is an anti-toxic gene; wherein the anti-toxic gene can be selected from any one of ccdA, ParD, MazE, Kis, HicB, RelB, VapB, Phd, RatB, HipB, Epsilon, ToxI, YeeU, CptB, GhoS, Sok, IstR-1, SymR, and PasB/C.

In some embodiments, the toxic gene is ccdB gene, and the toxic gene expression cassette comprises a promoter, a UTR sequence, and ccdB gene; the nucleic acid fragment 2 for transcribing the anti-toxic gene product is ccdA gene, and the anti-toxic gene expression cassette comprises araBAD promoter, arabinose operator, and ccdA gene.

The toxic gene and the anti-toxic gene can also be Zeta gene or Doc gene, and Epsilon gene or Phd gene, respectively.

In some embodiments, the toxic gene expression cassette comprises a promoter, a UTR sequence, and a Zeta gene gene product; the anti-toxic gene expression cassette comprises araBAD promoter, arabinose operator, and Epsilon gene. In some embodiments, the toxic gene expression cassette comprises a promoter, a UTR sequence, and a Doc gene gene product; the anti-toxic gene expression cassette comprises araBAD promoter, arabinose operator, and Phd gene.

In some specific embodiments, the UTR sequence is a 5'UTR sequence, and the 5'UTR sequence and the toxic gene are expressed in fusion, and the fused sequence is shown in SEQ ID NO: 1 or SEQ ID NO: 2:
SEQ ID NO:1 
gtttaaggaattttgtggctggccacgccgtaaggtggcaaggaactggttctgatgtggat-
ttacaggagccagaaaagcaaaaaccccgataatcttcttcaactttggcgagtacgaaaagattaccggggcccacttaaaccgt
atagccaacaattcagctatgcggggagtatagttatatgcccggaaaagttcaa-
gacttctttctgtgctcgctccttctgcgcattgtaagtgcaggatggtgtgcagtttaaggtttacacctataaaagagagagccgtta
tcgtctgtttgtggatgtacagagtgatattattgacacgcccgggcgac-
ggatggtgatccccctggccagtgcacgtctgctgtcagataaagtctcccgtgaactttacccggtggtgcatatcggggatgaaa
gctggcgcatgatgaccaccgatatggccagtgtgccggtctccgttatcggggaa-
gaagtggctgatctcagccaccgcgaaaatgacatcaaaaacgccattaacctgatgttctggggaatataa.
SEQ ID NO:2

ggtagacacacatcttgtcatatgatataatggtttcgccaaaaatcaataatcaga-
caacaaggtgcagtttaaggtttacacctataaaagagagagccgttatcgtctgtttgtggatgtacagagtgatattattgacacgc
ccgggcgacggatggtgatccccctggccagtgcacgtctgctgtcaga-
taaagtctcccgtgaactttacccggtggtgcatatcggggatgaaagctggcgcatgatgaccaccgatatggccagtgtgccgg

tctccgttatcggggaagaagtggctgatctcagccaccgcgaaaatgacatcaaaaac-
gccattaacctgatgttctggggaatataa.

In some specific embodiments, the nucleotide sequence of the anti-toxic gene expression cassette is shown in SEQ ID NO: 9.

SEQ ID NO: 9
gcccgtcacgggcttctcagggcgttttatggcgggtctgc-
tatgtggtgttatctgactttttgctgttcagcagttcctgccctctgattttccagtctgaccacttcggattatcccgtgacaggtcattc
agactggctaatgcacccagtaaggcagcgg-
tatcatcaacaggcttacccgtcttactgtctagactcgaggaattgatatcacccgacaccatcgaatggtcaccagtccctgttctc
gtcagcaaaagagccgttcatttcaataaaccgggcgacctcagccatcccttcctgat-
tttccgctttccagcgttcggcacgcagacgacgggcttcattctgcatggttgtgcttaccagaccggagatattgacatcatatgcc
ttgagcaactgatagctgtcgctgtcaactgtcactgtaatac-
gctgcttcatagtgaattcctcctgctagcccaaaaaaacgggtatggagaaacagtagagagttgcgataaaaagcgtcaggtag
gatccgctaatcttatggataaaaatgctatggcatagcaaagtgtgacgccgtgcaaa-
taatcaatgtggacttttctgccgtgattatagacacttttgttacgcgtttttgtcatggctttggtcccgctttgttacagaatgcttttaat
aagcggggttaccggtttggttagcgagaagagccagtaaaagacgcagtgacgg-
caatgtctgatgcaatatggacaattggtttcttctctgaatggcgggagtatgaaaagtatggctgaagcgcaaaatgatcccctgc
tgccgggatactcgtttaatgcccatctggtggcgggtttaacgccgattgaggccaac-
ggttatctcgatttttttatcgaccgaccgctgggaatgaaaggttatattctcaatctcaccattcgcggtcagggggtggtgaaaaat
cagggacgagaatttgtttgccgaccgggtgatattttgctgttcccgccaggagagat-
tcatcactacggtcgtcatccggaggctcgcgaatggtatcaccagtgggtttactttcgtccgcgcgcctactggcatgaatggctt
aactggccgtcaatatttgccaatacggggttctttcgcccggatgaagcgcaccagccg-
catttcagcgacctgtttgggcaaatcattaacgccgggcaaggggaagggcgctattcggagctgctggcgataaatctgcttga
gcaattgttactgcggcgcatggaagcgattaacgagtcgctccatccaccgatgga-
taatcgggtacgcgaggcttgtcagtacatcagcgatcacctggcagacagcaattttgatatcgccagcgtcgcacagcatgtttg
cttgtcgccgtcgcgtctgtcacatcttttccgccagcagttagggattagcgtcttaa-
gctggcgcgaggaccaacgtatcagccaggcgaagctgcttttgagcaccacccggatgcctatcgccaccgtcggtcgcaatg
ttggttttgacgatcaactctatttctcgcgggtatttaaaaaatgcac-
cggggccagcccgagcgagttccgtgccggttgtgaagaaaaagtgaatgatgtagccgtcaagttgtcataattggtaacgaatc
agacaattgacggcttgacggagtagcatagggtttgcagaatccctgcttcgtccattt-
gacaggcacattatgcatcttggagtggtgaatccgttagcgaggtgccgccggcttccat

In some specific embodiments, the nucleotide sequence of the anti-toxic gene expression cassette is shown in SEQ ID NO: 17 or 19.

In some specific embodiments, in the host cell expression cassette of the plasmid production system described in the present invention, the number of the anti-toxic gene expression cassettes and the number of the toxic gene expression cassettes are both selected from integers ≥ 1. Those skilled in the art can select an appropriate number based on the selected toxic gene and its toxicity and the inhibitory effect of the anti-toxic gene on the toxicity, so as to ensure that when the toxic gene product and the anti-toxic gene product are expressed simultaneously, the toxic effect of the toxic gene product is inhibited. In some specific embodiments, the number of the toxic gene expression cassette is 1, and the number of the anti-toxic gene expression cassette is specifically 1, 2, 3, 4 or more. In a specific embodiment, the toxic gene in the toxic gene expression cassette is ccdB gene, the anti-toxic gene in the anti-toxic gene expression cassette is ccdA gene, the number of the toxic gene expression cassette is 1, and the number of the anti-toxic gene expression cassette is 2.

In the present invention, according to the type of replication origin Ori, the host cell expression cassette may also be introduced with a Rep protein required for plasmid replication, such as R6K Rep, an essential protein for R6K plasmid replication, which is suitable for the preparation of plasmids such as R6K that require auxiliary proteins for conditional replication.

The plasmid described in the present invention comprises a nucleic acid fragment 3 for transcribing a small RNA; the sequence of the nucleic acid fragment 3 is selected from any one of the following a) to d):
a) a sequence shown in any one of SEQ ID NOs: 3 to 7;
b) a sequence in which one or more nucleotides are substituted, deleted or added to the sequence shown in a) and which has the same or similar function;
c) a sequence complementary to the sequence shown in a) or b);
d) a sequence that has at least 50% homology with the sequence shown in a), b) or c) and has unchanged function.

Wherein, the sequence shown in SEQ ID NO: 3 is:

agtcacaccatcctgcacttacaatgcgcagaaggagcgagcacagaaagaagtctt-
gaacttttccgggcatataactatactccccgcatagctgaattgttggctatacggtttaagtgggccccggtaatcttttcgtactcg

ccaaagttgaagaagattatcggggtttttgcttttctggctcctg-
taaatccacatcagaaccagttccttgccaccttacggcgtggccagccacaaaattccttaaac (VecSeqB1/258).

The sequence shown in SEQ ID NO:4 is:

agaaggagcgagcacagaaagaagtcttgaacttttccgggcatataactatactccccg-
catagctgaattgttggctatacggtttaagtgggccccggtaatcttttcgtactcgccaaagttgaagaagattatcggggtttttgct
tttctggctcctgtaaatccacatcagaaccagttccttgccaccttacggcgtggccagccacaaaattccttaaac
(VecSeqB1/229).

The sequence shown in SEQ ID NO:5 is:

agaaggagcgagcacagaaagaagtcttgaacttttccgggcatataactatactccccg-
catagctgaattgttggctatacggtttaagtgggccccggtaatcttttcgtactcgccaaagttgaagaagattatcggggtttttgct
tttctggctcctgtaaatccacatcagaac (VecSeqB1/181).

The sequence shown in SEQ ID NO:6 is:

agaaggagcgagcacagaaagaagtcttgaacttttccgggcatataactatactccccg-
catagctgaattgttggctatacggtttaagtgggccccggtaatcttttcgtactcgccaaagttgaagaagattatcggggtttttgct
ttt (VecSeqB1/154).

The sequence shown in SEQ ID NO:7 is:

cttttccgggcatataactatactccccgcatagctgaattgttggctatac-
ggtttaagtgggccccggtaatcttttcgtactcgccaaagttgaagaagattatcggggtttttgctttt (VecSeqB1/123).

In some embodiments, the nucleic acid fragment 3 can also be selected from the sequence shown in SEQ ID NO: 8:

Gtagaattggtaaagagagtcgtgtaaaatatcgagttcgcacatcttgttgtctgat-
tattgatttttggcgaaaccatttgatcatatgacaagatgtgtatctaccttaacttaatgattttgataaaaatcatta
(VecSeqB2/139).

In some embodiments, the nucleic acid fragment 3 can also be selected from the sequence shown in any one of SEQ ID NOs: 11, 13 and 15.

In some embodiments, the sequence of the nucleic acid fragment 3 of the present invention may have 50%, 55%, 60%, 65%, 70%, 75%, 80%, 85%, 90%, 95%, 98%, 99%, 100%, etc. homology with the sequence shown in any one of SEQ ID NOs: 3 to 8 or 11, 13, 15 or a complementary sequence thereof.

In some embodiments, the Ori replication origin is selected from ColE1, pBR322, pMB1, R6K, pUC, F1, p15A, 2µori or oriV.

The plasmid provided by the present invention may also comprise a target gene. Specifically, the plasmid may comprise a promoter, a target gene, an Ori replication origin, and a nucleic acid fragment 3 for transcribing a small RNA ligated in sequence.

In present invention, the promoter in the plasmid and the promoter in the toxic gene expression cassette can be a viral, prokaryotic and eukaryotic promoter or a synthetic promoter commonly used in the art, such as CMV, EF1A, EFS, CAG, CBh, SFFV, MSCV, SV40, mPGK, hPGK, UBC, RSV, Nanog, Nes, Tubala, Camk2a, SYN1, Hb9, Th, NSE, GFAP, Ibal, ProA1, hRK, hRHO, hBEST1, Prnp, Cnp, K14, BK5, mTyr, cTnT, aMHC, Myog, ACTA1, MHCK7, SM22a, EnSM22a, Runx2OC, Collal, Col2al, aP2, Adipoq, Tiel, Cd144, CD68, CD1b, Afp, Alb, TBG, MMTV, Wap, HIP, Pdxl, Ins2, Hcn4, NPHS2, SPB, CD144, TERT, TRE, TRE3G, GAL1, MET17, CUP1, AOX1, sCMV, bactin2, Ubi, cmlc2, zK5, 503unc, HSP70, 5 x UAS, CaMV35S, Nos, ZmUbi, TEF1, GPD, ADHl, GAP, actin5C, Polyubiquitin, a 1-tubulin, Rh2, Mtn, U6, U3, H1, U6-26, TK, RSV, MC1, GAL1, PH, p5, p10, p40, p41, araBAD, cspA, Hsp68, pL, pR, EM7, T7, SP6, AmpR promoter, etc., including but not limited to these.

In some embodiments, in the plasmid of the present invention, a polyA sequence may be added to the 3' end of the target gene. The plasmid comprises a promoter, a target gene, a polyA sequence, an Ori replication origin and a nucleic acid fragment encoding a small RNA inhibitory factor ligated in sequence.

In some embodiments, the resistance-free plasmid production system provided by the present invention also comprises a host, that is, the production system comprises host cell expression cassettes, a plasmid and a host, wherein the host is a host bacterium or a host cell, and the host bacterium and the host cell are derived from *Escherichia coli, Agrobacterium, Bacillus, Caulobacter* or a yeast. Those skilled in the art can select a suitable host type according to actual conditions, and the present invention has no special restrictions. In a specific embodiment of the present invention, the host is *Escherichia coli.* The host cell expression cassette of the present invention is integrated into the genome of the host bacterium or the host cell to obtain a strain integrated with the host cell expression cassette of the present invention. Based on this, the resistance-free production plasmid provided by the present invention comprises the above-mentioned strain and the above-mentioned plasmid.

The present invention also provides use of the plasmid production system in producing a plasmid or in preparing a product for cell gene therapy.

The present invention also provides a modified *Escherichia coli* strain, which has a genome in which the host cell expression cassette is integrated.

The *E. coli* may be a commonly used strain in the art, including DH5a, TOP10, NEB stable, BL21, EPI300, DH10B, JM109, SURE, Stbl3, XL10 and variants of the above strains. The integration site is a safe site of the *E. coli* genome, which can be common in the art.

Taking the arabinose operon as an example, the principle of plasmid screening by the resistance-free plasmid production system provided by the present invention is explained: when the strain is not added with arabinose, araC gene will inhibit the activity of araBAD promoter, and the anti-toxic gene cannot be expressed normally, while the toxic gene is expressed normally, and the strain dies; under the action of added arabinose, the anti-toxic gene can be expressed normally, thereby inhibiting the toxic gene, and the strain can grow normally. When the number of anti-toxic gene expression cassettes is ≥2, the survival rate of the strain is significantly increased, and the specific number is preferably 2, 3, 4, 5 or more .

When the host strain is transferred with the plasmid of the present invention, the small RNA transcribed in the plasmid binds to the UTR of the toxic gene in the host cell expression cassette through base complementarity, and the binding of the two can form a complex, and the resulting steric hindrance effect can effectively inhibit the translation of the toxic protein, thereby allowing the strain to survive. Specifically, experiments have shown that the steric hindrance effect generated by the binding of the small RNA transcribed in the plasmid to the UTR of the toxic gene can effectively control the expression of the toxic gene in the toxic gene product.

When the strain does not contain the plasmid of the present invention, due to the lack of arabinose in the culture medium, the expression of the anti-toxic protein cannot be activated, and the toxicity of the toxic protein cannot be inhibited. Therefore, the strain without the plasmid of the present invention will die during the culture process and cannot continue to divide.

The present invention also provides a method for producing a plasmid, wherein the plasmid in the plasmid production system of the present invention is transferred into a host integrated with the host cell expression cassette. In some specific embodiments, the transfer is carried out by chemical transfer or electroporation, and the specific steps are not limited, and can be carried out according to conventional steps in the art.

The present invention combines toxic gene products/antitoxic gene products, operon-related elements and small RNAs to construct a novel resistance-free plasmid production system, which comprises host cell expression cassettes and a plasmid. The expression of the toxic gene in the modified strain is controlled by binding of the small RNA sequence in the plasmid with the toxic gene product in the host cell expression cassette, without the need for antibiotics and other auxiliary inhibitors. The resistance-free backbone can be combined with various types of plasmid replication origins (Ori) to form a novel resistance-free plasmid production system. Compared with the prior art, the present invention has the following advantages:
1. The entire plasmid production process does not require the addition of antibiotics or other auxiliary inhibitors, which solves the harm of antibiotics and inhibitors to plasmids in gene therapy and reduces the cost of plasmid preparation and purification.
2. According to different Ori lengths and replication characteristics, the prokaryotic backbone sequence can be greatly shortened, and the copy number of some plasmids can reach 4-5 times when compared with the conventional plasmid, which improves application safety and greatly reduces production costs.
3. The plasmid backbone provided by the present invention only comprises Ori required for self-replication and a small RNA inhibitor, and the remaining sequences are for target gene expression. The prokaryotic backbone region of the traditional plasmid is between 2000-2500bp, which is usually 4-5 times of the plasmid of the present invention. The proportion of the prokaryotic backbone region of the plasmid of the present invention is greatly reduced, solving the problem that the sequence outside the target gene expression cassette accounts for too large a proportion in the plasmid and the yield cannot be increased.

### BRIEF DESCRIPTION OF THE DRAWINGS

Figure 1 shows an example map of the functional elements of the miniVec stable strain;
Figure 2 shows the map of miniVec Plasmid;
Figure 3 shows a diagram of the synergistic effect of VecSeqA/VecSeqB and araBAD-ccdB/cddA;
Figure 4 shows the map of Intermediate Vector 1;
Figure 5 shows the map of Intermediate Vector 2;
Figure 6 shows the map of pHelper Vector1;
Figure 7 shows the map of pHelper Vector2;
Figure 8 shows the map of Intermediate Vector 3;
Figure 9 shows the map of Intermediate Vector4;
Figure 10 shows the map of Intermediate Vector5;
Figure 11 shows the map of Intermediate Vector6;
Figure 12 shows the map of Intermediate Vector7;
Figure 13 shows the map of Intermediate Vector8.

### DETAILED DESCRIPTION

The present invention provides a resistance-free plasmid production system and the use thereof. Those skilled in the art can refer to the content of this application and appropriately improve the process parameters to achieve it. It is particularly important to point out that all similar replacements and modifications are apparent to those skilled in the art, and they are all considered to be comprised in the present invention.

The present invention constructs a novel resistance-free plasmid production system and the use thereof. The system combines toxic gene products/anti-toxic gene products, operon-related elements and small RNAs to construct a novel resistance-free plasmid production system. The system comprises host cell expression cassettes and a plasmid. The expression of the toxic gene in the strain integrated with the host expression cassette is controlled by the transcribed small RNA sequence of the plasmid, without the need to use antibiotics and other auxiliary inhibitors. The resistance-free backbone can be combined with various types of plasmid replication origin (Ori) to form a novel resistance-free plasmid production system. Furthermore, according to different Ori lengths and replication characteristics, the prokaryotic backbone sequence can be greatly shortened, and the plasmid copy number can reach 4-5 times when compared with the conventional plasmid, which improves the application safety while greatly reducing the production cost.

The definitions of terms used in this application are as follows:
"Gene product" refers to a single-stranded ribonucleic acid (RNA) product synthesized by DNA gene production, which can produce various mature RNA products after processing, such as non-coding RNAs such as mRNA, tRNA or rRNA, etc..
"Transcribing a toxic gene product" and "a toxic gene product" can be used interchangeably, and refer to a gene product expressing toxic substances that are toxic to organisms and prevent the organisms from growing normally. Toxic genes that can produce toxic gene products of the present invention are known in the art, for example, selected from any one of ccdB, ParE, MazF, Kid, HicA, RelE, VapC, Doc, RatA, HipA, Zeta, ToxN, YeeV, CptA, GhoT, Hok, TisB, SymE, and PasA, including but not limited to these.

"Transcribing an anti-toxic gene product" and "anti-toxic gene product" can be used interchangeably and refer to a gene product expressing anti-toxic substances that can resist toxicity. Anti-toxic genes that can produce the anti-toxic gene products of the present invention are known in the art. For example, the anti-toxic gene is selected from any one of ccdA, ParD, MazE, Kis, HicB, RelB, VapB, Phd, RatB, HipB, Epsilon, ToxI, YeeU, CptB, GhoS, Sok, IstR-1, SymR, and PasB/C, including but not limited to these.

"A nucleic acid fragment for transcribing a small RNA" refers to an expression cassette that transcribes a small RNA, including a promoter, a non-coding RNA, and a terminator. "A small RNA inhibitor" and "a small RNA" can be used interchangeably and refer to a non-coding RNA that is reversely complementary to the toxic gene product of the present invention to produce steric hindrance effect. The length of the non-coding RNAs that are usually expressed is less than 300 nucleotide sequences.

"Steric effect " refers to the complementary binding of a RNA sequence with a DNA or a RNA target sequence to form a double-stranded complex, which changes the spatial structure of the target sequence, making it impossible for the target sequence to effectively bind to enzymes related to replication, gene production or translation, thereby affecting the replication and expression of the target sequence itself.

"UTR" is an untranslated region, located at the 5' end or 3' end of mature mRNA, which does not encode protein, but has a translation regulatory function, and participates in regulating the stability and intracellular localization of a mRNA. UTR sequences that can form a hairpin loop or can interact with a stem-loop structure are all suitable for the present invention. UTR sequences can be selected from RNAII of ColEI, repA of IncF, hok of IncFII, repZ of IncI and ColIb, rep of CoIE2, rep1 of R1162, repR of pIP501, repC of pT181, repB of IncB and pLS1, Q and cII of lambda, ant of P22, P1 and P7, tnp of IS10, ompF, crp, fhlA, pks12, dinQ, tatC, bsrG, bsrE, RalR and rpoS of bacteria, etc., including but not limited to these. Once the UTR sequence is determined, a small RNA inhibitor that is reversely complementary to it and produces a steric hindrance effect can be designed according to conventional techniques.

"Ori replication origin" refers to the origin of replication of the plasmid.

"Rep protein" refers to a class of proteins that act on the replication origin of the plasmid and control plasmid replication.

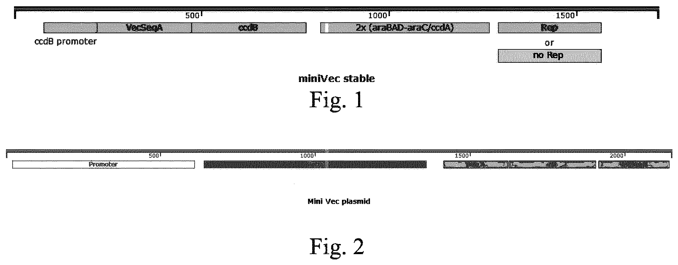

In a specific example, the nucleic acid fragment 1 for transcribing the toxic gene product of the present invention comprises a UTR sequence (labeled as VecSeqA) and a toxic gene ccdB, the nucleic acid fragment 2 for transcribing the anti-toxic gene product is the anti-toxic gene ccdA, and the nucleic acid fragment 3 for transcribing the small RNA in the plasmid is labeled as VecSeqB. Taking this as an example, the resistance-free plasmid production system of the present invention is described in detail as follows:
1. miniVec stable strain
   (1) The ccdB/ccdA system is used to modify the E. coli genome, that is, a ccdB toxic gene expression cassette and two tandem araBAD-araC/ccdA anti-toxic gene expression cassettes are inserted into the safe sites of the *E. coli* strain genome. Without the addition of arabinose, the araC gene will inhibit the activity of the araBAD promoter, and the ccdA anti-toxic gene cannot be expressed normally, the ccdB toxic gene is expressed normally, and the strain will die; with the addition of arabinose, the anti-toxic gene ccdA can be expressed normally, thereby inhibiting the ccdB toxic gene, and the strain can grow normally.
   (2) A 5'UTR sequence (VecSeqA for short) is inserted before the ccdB start codon. This sequence can be bound by the small RNA inhibitor in the miniVec, so that in the absence of arabinose, as long as the strain contains the miniVec plasmid and provides the small RNA inhibitor to inhibit the expression of the ccdB toxic gene, the strain can survive. Further, high number of copies of plasmids can be extracted from the surviving strain. (3) Optionally, depending on the type of replication origin Ori, a Rep protein, such as the R6K plasmid replication-essential protein R6K Rep can be introduced into the genome of the *E. coli* strain, which is suitable for the preparation of plasmids such as R6K that require auxiliary proteins for conditional replication. An example of functional elements of the strain (i.e., host cell expression cassette) is shown in Figure 1.

### (II) miniVec resistance-free plasmid

It only comprises Ori required for self-replication of the plasmid and nucleic acid fragment 3 (named VecSeqB) for transcribing a small RNA inhibitor, and the rest of the sequence is the target gene expression cassette (shown in Figure 2). The prokaryotic backbone region of traditional plasmids is between 2000-2500bp, and according to different Ori selections, the proportion of the prokaryotic backbone region of miniVec Plasmid in the plasmid can be greatly reduced, usually reducing the sequence length by 4-5 times.

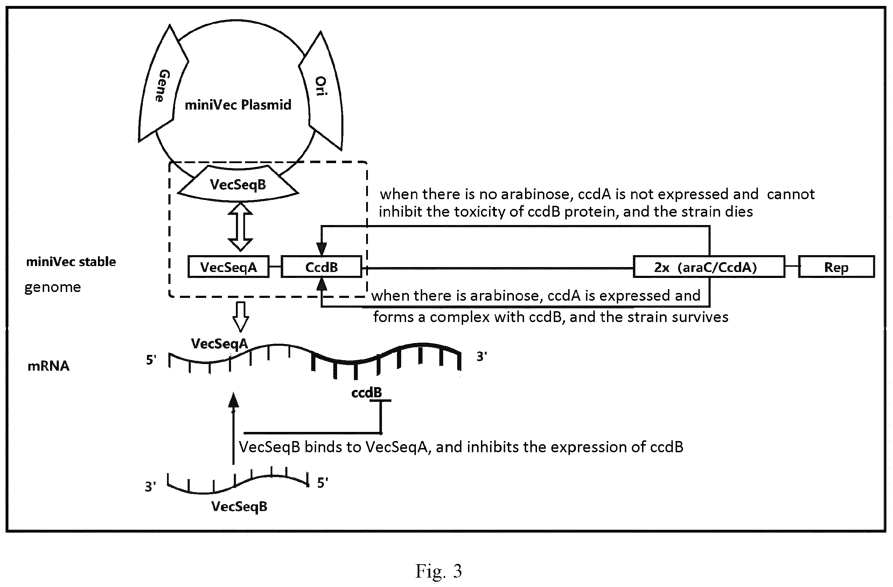

The synergistic effect of VecSeqB with VecSeqA/ccdB and araBAD-araC/ccdA is shown in Figure 3. When the miniVec stable strain contains miniVec Plasmid, VecSeqA binds to VecSeqB to form a complex, which causes a steric effect to inhibit the translation of ccdB protein, allowing the bacteria to survive; when the miniVec stable strain does not contain miniVec Plasmid, due to the lack of arabinose in the culture medium, the expression of ccdA protein cannot be activated, and the toxicity of ccdB protein cannot be inhibited, so the bacteria without miniVec Plasmid will die during the culture process and cannot continue to divide. The entire plasmid production process does not require the addition of antibiotics or other auxiliary inhibitors, which solves the harm of antibiotics and inhibitors to plasmids in gene therapy, and can also reduce the cost of plasmid preparation and purification.

The test materials used in the present invention are all common commercial products and can be purchased in the market.

The present invention will be further described below in conjunction with the Examples.

### Example 1

Modification of MiniVec stable strain:
miniVec stable strain 1: A VecSeqA1/ccdB protein expression cassette (i.e., toxic gene expression cassette), an araC/ccdA protein expression cassette (anti-toxic gene expression cassette), and a R6K Rep protein expression cassette were inserted into DH5a strain. The VecSeqA1 sequence was selected from repA of IncF.
miniVec stable strain 2: A VecSeqA1/ccdB protein expression cassette, two tandem araC/ccdA protein expression cassettes, and a R6K Rep protein expression cassette were inserted into DH5a strain.

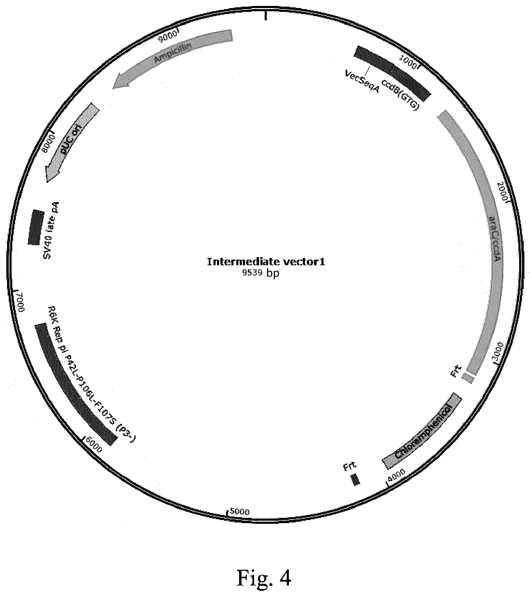

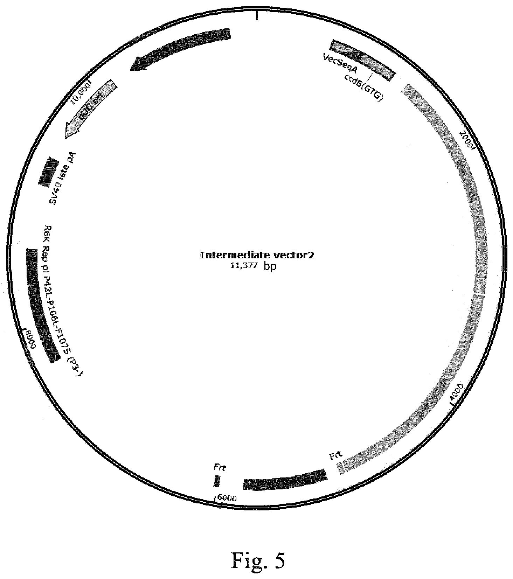

Construction of Intermediate Vector (the intermediate vector comprises a homology arm HA that recombines with the bacterial genome):
Intermediate Vector 1: expressing VecSeqA1/ccdB protein (SEQ ID NO: 1, wherein positions 1-252 are UTR), an araC/ccdA protein, and a R6K Rep protein, as shown in Figure 4;
Intermediate Vector 2: expressing VecSeqA1/ccdB protein (SEQ ID NO: 1, wherein positions 1-252 are UTR), two tandem araC/ccdA proteins, and a R6K Rep protein, as shown in Figure 5.

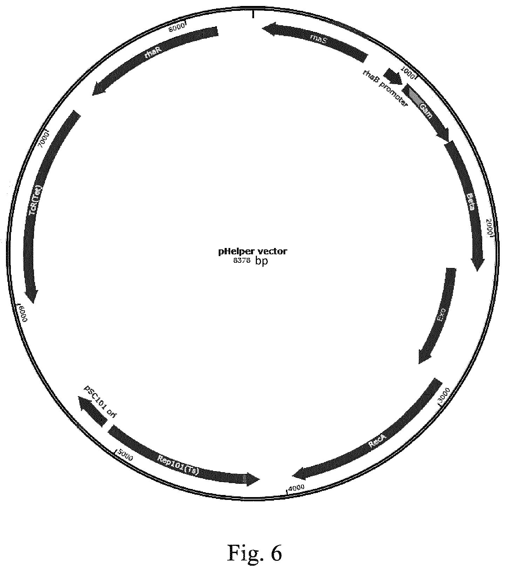

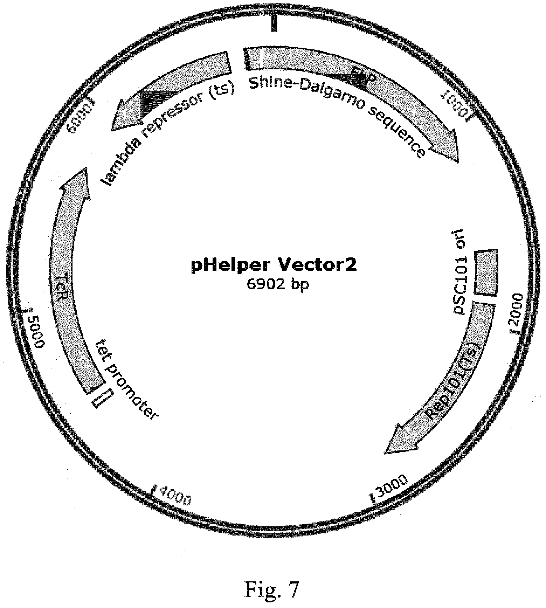

Construction of temperature-sensitive helper plasmid (pHelper Vector):
pHelper Vector1: A 30°C temperature-sensitive helper plasmid that expresses pRed/ET homologous recombinase induced by rhamnose. This plasmid is Tet-resistant, as shown in Figure 6.
pHelper Vector2: A 30°C temperature-sensitive helper plasmid that expresses Flp/Frt site recombinase induced by arabinose. This plasmid is Tet-resistant, as shown in Figure 7.

Intermediate Vector 1 and Intermediate Vector 2 were digested with AarI and the recovered recombinant fragments were as follows:
Recombinant fragment 1: DH5a HA-VecSeqA1/ccdB-araC/ccdA-Frt-Chl-Frt-R6K Rep-DH5a HA, 7195bp;
Recombinant fragment 2: DH5a HA-VecSeqA1/ccdB-2(araC/ccdA)-Frt-Chl-Frt-R6K Rep-DH5a HA, 9033bp;

DH5a strain was electroporated with 1 µg pHelper vector1, coated on Tet plate, and cultured overnight at 30°C. Single clones were picked the next day, and colony PCR was performed to identify whether pHelper Vector had entered the DH5a strain. Positive clones were retained and called Intermediate Strain, which was used as the strain for the next experiment.

Preparation of Intermediate Strain electroporation competent cells:
1 mL of Intermediate Strain bacterial solution was inoculated into 100 mL of Tet LB medium and cultured at 30°C until OD600 was 0.2-0.3. 2 mL of 10% rhamnose was added and the cells were cultured at 37°C for 45 min to induce pHelper Vecto1 to express the recombinase. At the same time, pHelper vector1 was removed and the Intermediate Strain electroporation competent cells were prepared according to the conventional steps.

Intermediate Strain competent cells were electroporated with 1 µg of recombinant fragment 1 and recombinant fragment 2 recovered in 1.3 respectively, coated on Chl+Tet+0.37% arabinose plate, and cultured at 37°C overnight. At this time, the DH5α genome homology arms on recombinant fragments 1 and 2 were recombined with the DH5α genome under the action of recombinase, and VecSeqA1/ccdB-araC/ccdA-Frt-Chl-Frt-R6K Rep or VecSeqA1/ccdB-2(araC/ccdA)-Frt-Chl-Frt- R6K Rep was inserted into the DH5α genome, and the following initial strains were obtained respectively: miniVec stable strain 1 and miniVec stable strain 2.

Colony PCR was performed to identify whether miniVec stable strain 1 and miniVec stable strain 2 were respectively inserted with the recombinant target fragment. The positive clones of colony PCR were sent for sequencing to determine the integrity of the recombinant target fragment. The sequencing results were as follows:
miniVec stable strain 1: In VecSeqA 1/ccdB-araC/ccdA-Frt-Chl-Frt-R6K Rep target fragment, ccdB sequence was mutated, making the ccdB protein non-toxic. That is, the ccdA protein expressed by one araC/ccdA expression cassette was not enough to inhibit the toxicity of the ccdB protein, so that the strain itself mutated and gained viability, which was related to the use of a stronger promoter to mediate the expression of the toxic gene in Example 1 of the present invention.
miniVec stable strain 2: In VecSeqA1/ccdB-2(araC/ccdA)-Frt-Chl-Frt-R6K Rep target fragment, the ccdB sequence was intact without mutation or deletion, and the ccdB protein maintained its toxic function; this indicated that the ccdA protein expressed by the two araC/ccdA expression cassettes were sufficient to inhibit the toxicity of the ccdB protein, allowing the strain to grow normally. At this time, the strain was called: miniVec stable-Chl-A1.
miniVec stable-Chl-A1electroporation competent cells were prepared according to conventional procedures.
miniVec stable-Chl-A1 competent cells were electroporated with 1 µg of pHelper Vector2 comprising Flp recombinase, coated on 0.37% arabinose plate, and cultured at 30°C overnight.

The strain with the Chl resistance gene deleted after Frt recombination was screened, and the positive clones were sent for sequencing to verify the integrity of the fragment VecSeqA1 /ccdB-2(araC/ccdA)-Frt-R6K Rep. The pHelper vector2 was removed by culturing at 37°C. This strain was the miniVec stable -A1 strain.

### Construction of miniVec Plasmid

VecSeqB 1 was designed with multiple versions of sequences. According to the differences of Ori and VecSeqB, the different miniVec Plasmids shown in Table 1 were constructed using Gibson homologous recombination reaction:

**Table 1 Summary of different minivec Plasmids**

| Serial number | Vector Name | VecSeqB length | VecSeqB sequences |
|---|---|---|---|
| miniVec Plasmid 1 | pR6K-VecSeqB1/₂₅₈-EGFP | 258bp | SEQ ID NO:3 |
| miniVec Plasmid 2 | pR6K-VecSeqB1/₂₂₉ - EGFP | 229bp | SEQ ID NO:4 |
| miniVec Plasmid 3 | pR6K-VecSeqB1/₁₈₁-EGFP | 181bp | SEQ ID NO:5 |
| miniVec Plasmid 4 | pR6K-VecSeqB1/₁₅₄-EGFP | 154bp | SEQ ID NO:6 |
| miniVec Plasmid 5 | pR6K-VecSeqB1/₁₂₃-EGFP | 123bp | SEQ ID NO:7 |
| miniVec Plasmid 6 | pUC-VecSeqB1/₂₅₈-EGFP | 258bp | SEQ ID NO:3 |

Among them, in the miniVec Plasmid 2, the length of the VecSeqB 1 sequence is 229 bp, and its sequence is shown in SEQ ID NO: 4, that is, the nucleotide sequence shown in positions 30 to 258 of SEQ ID NO: 3.

In the miniVec Plasmid 3, the length of the VecSeqB 1 sequence is 181 bp, and its sequence is shown in SEQ ID NO: 5, that is, the nucleotide sequence shown in positions 30 to 210 of SEQ ID NO: 3.

In the miniVec Plasmid 4, the length of the VecSeqB 1 sequence is 154 bp, and its sequence is shown in SEQ ID NO: 6, that is, the nucleotide sequence shown in positions 30 to 183 of SEQ ID NO: 3.

In the miniVec Plasmid 5, the length of the VecSeqB 1 sequence is 123 bp, and its sequence is shown in SEQ ID NO: 7, that is, the nucleotide sequence shown in positions 61 to 183 of SEQ ID NO: 3.

Test of the synergistic effect of VecSeqA1/VecSeqB1 and miniVec stable-A1 strain
miniVec stable-A1 strain was electroporated with 100ng miniVec Plasmids 1-6 respectively, coated on conventional antibiotic free plate, and cultured at 37°C overnight. The results are shown in Table 2. As can be seen from Table 2, the miniVec strain in Group 1 did not comprise miniVec Plasmid, and the number of clones on the antibiotic free plate was 2.99E+05 times lower than that on the plate with arabinose, indicating that in the absence of arabinose, araC could inhibit ccdA expression, and ccdB protein killed the strain, and the number of clones was significantly reduced. In Groups 2, 3, 4 and Group 7, miniVec Plasmid was transferred into the miniVec strain, and the number of clones on the antibiotic free plate increased by 2.07E+03-1.27E+04 times compared with the antibiotic free plate in Group 1, indicating that the combination of VecSeqB 1 (SEQ ID NO: 3~5) with VecSeqA 1 (SEQ ID NO: 1) could effectively inhibit the expression of ccdB protein. Groups 2, 3, 4 and Group 7 were miniVec Plasmids with different Ori, and their screening results were not much different, indicating that the miniVec strain could be used for preparation of plasmids with various types of Ori such as R6K and pUC. In the data of Group 5 and Group 6, miniVec Plasmid 4 and 5 were transferred into miniVec stable-A1 strain, and the number of clones on the antibiotic free plate increased by 1-5 times compared with the antibiotic free plate of Group 1, indicating that different truncated versions of VecSeqB 1 sequences had different binding efficiencies with VecSeqA1. Since the sequences of VecSeqB1/₁₅₄ and VecSeqB1/₁₂₃ were shorter, the expression efficiency of inhibiting ccdB toxic protein was lower than that of other sequences, but still had a certain screening effect.

For ease of understanding, the folds of screening effect in Table 2 were converted into a more easily interpreted plasmid screening positive rate (see the rightmost column), and the calculation formula was: Plasmid screening positive rate = (1-negative rate) x 100%, where negative rate = number of clones on antibiotic free plate of only miniVec strain/number of clones on antibiotic free plate of the corresponding group. From the results, it can be seen that without adding antibiotics and auxiliary agents, the plasmid screening positive rate ranges from 20% to 100%, reaching the level of conventional antibiotic plasmid screening. The plasmid screening positive rate of Group 1 is 0% because there is no miniVec Plasmid described in the present invention in the strain. The small RNA sequences in the Plasmids in Groups 3-6 are step-by-step truncated versions of the small RNA sequence of the Plasmid in Group 2, indicating that sequences with certain similarities can still produce a steric effect and play a role in plasmid screening.

**Table 2 Summary table of data of transformation effect**

| Group | | Number of clones per 1mL transformation bacterial solution | | Screening effect (folds) | Plasmid screening positive rate |
|---|---|---|---|---|---|
| | | 0.37% arabinose plate | Antibiotic free conventional plate | | |
| Group 1 | only minivec strain | 8.36E+08 | 2.80E+03 | 2.99E+0 5 | 0% |
| Group 2 | minivec strain + minivec Plasmid 1 | / | 9.35E+06 | 3.34E+0 3 | ≈100% |
| Group 3 | minivec Strain + minivec Plasmid 2 | / | 1.22E+07 | 4.37E+0 3 | ≈100% |
| Group 4 | minivec Strain + minivec Plasmid 3 | / | 5.79E+06 | 2.07E+0 3 | ≈100% |
| Group 5 | minivec Strain + minivec Plasmid 4 | / | 1.26E +0 4 | 4.50 | 78% |
| Group 6 | minivec Strain + minivec Plasmid 5 | / | 3.50 E+0 3 | 1.25 | 20% |
| Group 7 | minivec Strain + minivec Plasmid 6 | / | 3.56E+07 | 1.27E+0 4 | ≈100% |
| Group 8 | only minivec strain | 7.74EE+08 | 2.00E+03 | 3.87 E+05 | 0% |
| Group 9 | minivec Strain + minivec Plasmid 7 | / | 2.96E+06 | 1.48E+0 3 | ≈100% |

ccdB gene sequencing verification:
The clones of Group 2-Group 4, as well as 3 clones picked from the 0.37% arabinose plate and the antibiotic free plate of Group 1, were sent for sequencing to verify the integrity of the ccdB element. The sequencing results are summarized in Table 3:

**Table 3 Summary of sequencing results of ccdB gene sequence integrity**

| Group | | Sequencing results | | |
|---|---|---|---|---|
| | | Clone 1 | Clone 2 | Clone 3 |
| Group 1 | only miniVec -A1 strain (antibiotic free plate) | ccdB- | ccdB* | ccdB- |
| | only minivec -A1 strain (0.37% arabinose plate) | ccdB+ | ccdB+ | ccdB+ |
| Group 2 | minivec -A1 strain + minivec Plasmid 1 | ccdB+ | ccdB+ | ccdB+ |
| Group 3 | minivec -A1 strain + minivec Plasmid 2 | ccdB+ | ccdB+ | ccdB+ |
| Group 4 | minivec -A1 strain + minivec Plasmid 3 | ccdB+ | ccdB+ | ccdB+ |
| Notes: | | | | |
| ccdB- : ccdB sequence deleted; ccdB* : ccdB sequence mutation; ccdB+ : complete ccdB sequence, without deletion or mutation | | | | |

As can be seen from Table 3, the ccdB sequence of the clones on the antibiotic free plate of Group 1 had varying degrees of deletion or mutation, indicating that in the absence of arabinose, the ccdA protein could not be expressed, and the strain could only rely on its own mutations or deletions of ccdB element sequence to lose ccdB toxicity so as to survive. The sequencing results of arabinose plate clones showed that the ccdB sequences was normal, so arabinose could activate ccdA protein expression and kept the strain alive. In Group 2-Group 4, with the addition of miniVec Plasmid, no mutations or deletions were found in the ccdB sequence even in the absence of arabinose, indicating that the binding of VecSeqB 1 and VecSeqA 1 could significantly and effectively inhibit the expression of ccdB protein.

Extraction of miniVec Plasmid DNA, and comparison of yields of different miniVec Plasmids:
Three clones from antibiotic free plates of Group 2-Group 7 were selected, inoculated into 5 mL LB medium, and cultured at 37°C overnight.

The yields are shown in Table 4, and the traditional strain DH5a and the traditional resistance plasimd, pAAV[Exp]-CMV>EGFP (https://www.vectorbuilder.cn/vector/VB191212-1252ttc.html) were used as a control.

**Table 4 summary of the yields of MiniVec Plasmids**

| Group | | Yield ( µg /mL) | | |
|---|---|---|---|---|
| | | Clone1 | Clone2 | Clone3 |
| control | Traditional strain DH5α + traditional resistance plasmid pAAV[Exp]-CMV>EGFP | 3.00 | 3.08 | 3.33 |
| Group 2 | minivec -A1 strain + minivec Plasmid 1 | 8.39 | 9.28 | 8.64 |
| Group 3 | minivec -A1 strain + minivec Plasmid 2 | 8.69 | 9.21 | 7.30 |
| Group 4 | minivec -A1 strain + minivec Plasmid 3 | 7.19 | 6.23 | 7.18 |
| Group 5 | minivec -A1 strain + minivec Plasmid 4 | 1.74 | 0.41 | 1.98 |
| Group 6 | minivec -A1 strain + minivec Plasmid 5 | 0.39 | 0.40 | 1.83 |
| Group 7 | minivec -A1 strain + minivec Plasmid 6 | 7.19 | 6.23 | 7.18 |

As can be seen from Table 4, Group 5 and Group 6 could not achieve highly efficient screening due to the mutation of ccdB itself (which made the strains survive). Thus, the number of plasmid copies entering into the corresponding strains was significantly reduced. On the contrary, compared with the yields of traditional resistance plasmids, the yields of miniVec plasmids in Group 2 -Group 4 and Group 7 were increased by about 2-3 times, and the plasmid yields of miniVec Plasmids of different Ori (miniVec Plasmid 1 and miniVec Plasmid 6) were not much different, indicating that the miniVec strain could be applied to the preparation of various types of plasmids such as R6K and pUC plasmids. In the case of expressing the same target gene, the total length of the traditional plasmid sequence was about 2 times longer than that of miniVec Plasmid, that is, for the same weight of plasmid, the copy number of miniVec Plasmid was twice that of the traditional plasmid, which means that the copy number of the plasmids of the target gene produced by some strains and plasmids of the present invention could be increased by 4-6 times.

As can be seen from the above Example, different clones have different sequence lengths, different steric hindrance efficiencies, and different toxic protein inhibition efficiencies, which in turn lead to different plasmid yields. However, each group is able to produce positive resistance-free clones. Even if the yields of few groups are low, they can still solve the technical problem faced by the present invention, that is, to achieve the screening and production of resistance-free plasmids without adding antibiotics and other auxiliary agents. In other words, as long as the screening and production of resistance-free plasmids can be achieved without adding antibiotics and other auxiliary inhibitors, the bottleneck problem of the prior art that antibiotics or other auxiliary inhibitors must be added can be solved, and the level of plasmid yield can be solved by further optimizing the specific small RNA sequences.

### Example 2

The 5'UTR of the toxic gene was replaced with VecSeqA2 of another sequence (the nucleic acid sequence shown in positions 1 to 64 of SEQ ID NO: 2, the sequence was selected from tnp of IS10), and the sequence on the miniVec vector was designed to be VecSeqB2 (SEQ ID NO: 8) to further test the effect.

MiniVec stable strain modification:
miniVec stable strain 3: A VecSeqA2/ccdB protein expression cassette, two araC/ccdA protein expression cassettes, and a R6K Rep protein expression cassette were inserted into DH5a strain.

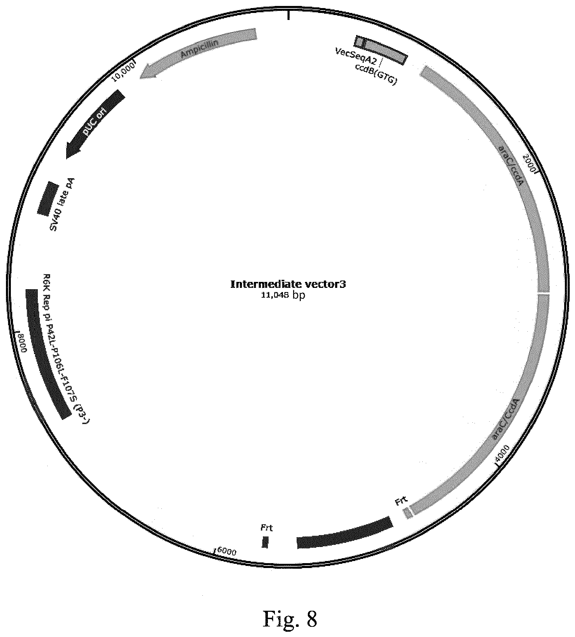

Construction of Intermediate Vector (the intermediate vector comprises a homology arm HA that recombines with the bacterial genome):
Intermediate Vector 3: expressing VecSeqA2/ccdB protein (SEQ ID NO: 2), two araC/ccdA proteins, and R6K Rep protein, as shown in Figure 8;
Construction of temperature-sensitive helper plasmid (pHelper Vector):
   Same as 1.2 of Example 1.
Intermediate Vector 3 was digested with AarI and the recovered recombinant fragments were as follows:
   Recombinant fragment 3: DH5a HA-VecSeqA2/ccdB-araC/ccdA-Frt-Chl-Frt-R6K Rep-DH5α HA, 8704 bp, as shown in Figure 8;
   DH5a strain was electroporated with 1 µg pHelper vector1, coated on Tet plate, and cultured overnight at 30°C. Single clones were picked the next day, and colony PCR was performed to identify whether pHelper Vector had entered the DH5a strain. Positive clones were retained and called Intermediate Strain, which was used as the strain for the next experiment.

Preparation of Intermediate Strain electroporation competent cells:
1 mL of Intermediate Strain bacterial solution was inoculated into 100 mL of Tet LB medium and cultured at 30°C until OD600 was 0.2-0.3. 2 mL of 10% rhamnose was added and the cells were cultured at 37°C for 45 min to induce pHelper Vecto1 to express the recombinase. At the same time, pHelper vector1 was removed and the Intermediate Strain electroporation competent cells were prepared according to the conventional steps.

Intermediate Strain competent cells were electroporated with 1 µg of recombinant fragment 3 recovered in 1.3, coated on Chl+Tet+0.37% arabinose plate, and cultured overnight at 37°C. At this time, the DH5a genome homology arm on the recombinant fragment 3 was recombined with the DH5a genome under the action of the recombinase, and VecSeqA2 /ccdB-araC/ccdA-Frt-Chl-Frt-R6K Rep was inserted into the DH5a genome, and the initial strain was obtained: miniVec stable strain 3.

Colony PCR was performed to identify whether the miniVec stable strain 3 was inserted with the recombinant target fragment. The positive clones of colony PCR were sent for sequencing to determine the integrity of the recombinant target fragment. The sequencing results were as follows:
miniVec stable strain 3: In VecSeqA2/ccdB-2(araC/ccdA)-Frt-Chl-Frt-R6K Rep target fragment, the ccdB sequence was intact without mutation or deletion, and the ccdB protein maintained its toxic function; this once again shows that the ccdA protein expressed by the two araC/ccdA was sufficient to inhibit the toxicity of the ccdB protein, allowing the strain to grow normally. At this time, the strain was called: miniVec stable-Chl-A2.

MiniVec stable-Chl-A2 electroporation competent cells were prepared according to conventional steps.

miniVec stable-Chl-A2 competent cells were electroporated with 1 µg of pHelper Vector2 comprising Flp recombinase, coated on 0.37% arabinose plates, and cultured at 30°C overnight.

The strain with the Chl resistance gene deleted after Frt recombination was screened, and the positive clones were sent for sequencing to verify the integrity of the fragment VecSeqA2/ccdB-2(araC/ccdA)-Frt-R6K Rep. The pHelper vector2 was removed by culturing at 37°C. This strain was miniVec stable -A2 strain.

### Construction of miniVec Plasmid

miniVec Plasmid 7 in Table 5 was constructed using the Gibson homologous recombination reaction:

**Table 5 information table of miniVec Plasmid 7**

| Serial number | Vector Name | VecSeqB length | VecSeqB sequence |
|---|---|---|---|
| miniVec Plasmid 7 | pR6K-VecSeqB2/₁₃₉ -EGFP | 139 bp | SEQ ID NO:8 |

### Test of the synergistic effect of VecSeqA2/VecSeqB2 and miniVec stable-A2 strain

miniVec stable-A2 strain was electroporated with 100 ng of miniVec Plasmid 7, coated on conventional antibiotic free plate, and cultured at 37°C overnight. The results are shown in Table 6. As can be seen from Table 6, the miniVec strain in Group 8 did not contain miniVec Plasmid, and the number of clones on the antibiotic free plates was 3.87 E+05 times lower than that on the plate with arabinose, which again showed that in the absence of arabinose, araC could inhibit the expression of ccdA, and the ccdB protein killed the strain, and the number of clones was significantly reduced. In Group 9, miniVec Plasmid 7 was transferred into miniVec stable-A2 strain, and the number of clones on the antibiotic free plate increased by 1.48 E+0 3 times compared with that on the antibiotic free plate of Group 8, and the Plasmid screening positive rate was almost 100%, indicating that the partial complementary binding of VecSeqB2 ( SEQ ID NO: 8) and VecSeqA2 (the nucleic acid sequence shown in positions 1 to 64 of SEQ ID NO: 2) could effectively inhibit the expression of ccdB protein; the VecSeqA sequences of miniVec-A2 strain and miniVec-A1 strain are completely different, but as long as the VecSeqB sequence on the miniVec vector can produce a sequence complementary to it, the inhibitory effect on ccdB is still effective, which shows that different sequence versions of 5'UTR can be inserted before the ccdB start codon, and the screening effect can be achieved by designing a VecSeqB that is complementary to it.

**Table 6 Summary table of data of transformation effect of MiniVec Plasmid 7**

| Group | | Number of clones per 1mL transformation bacterial solution | | Screening effect (folds) | Plasmid screening positive rate |
|---|---|---|---|---|---|
| | | 0.37% arabinose plate | Antibiotic free conventional plate | | |
| Group 8 | only miniVec -A2 strain | 7.74E+ 08 | 2.00E+03 | 3.87E+05 | 0% |
| Group 9 | minivec -A2 strain + miniVec Plasmid 7 | / | 2.96E+06 | 1.48E+03 | ≈100% |

Three clones were picked from the Group 9 antibiotic free plate, inoculated into 5 mL of LB medium, and cultured overnight at 37C. The yields are shown in Table 7.

**Table 7 summary of the yields of MiniVec Plasmid 7**

| Group | | Yield ( µg /mL) | | |
|---|---|---|---|---|
| | | Clone1 | Clone2 | Clone3 |
| Group 9 | miniVec-A2 strain + miniVec Plasmid 7 | 4.40 | 1.89 | 0.76 |

From the yields in Table 7, it can be seen that the combination of VecSeqB2 and VecSeqA2 can inhibit the expression of ccdB protein and achieve plasmid screening; however, the yields of different clones are different.

The results show that plasmid screening can be achieved as long as the RNA sequence of the miniVec vector is partially or completely complementary to the toxic gene product.

Example 3 Testing of various nucleic acid fragments 1 and 3 producing steric hindrance effects

The UTR sequence of nucleic acid fragment 1 was changed to VecSeqA3, VecSeqA4, and VecSeqA5, which were selected from rep of CoIE2, repC of pT181, and hok of IncFII, respectively. The sequence of nucleic acid fragment 3 was designed to be the corresponding VecSeqB3, VecSeqB4, and VecSeqB5 respectively. The specific sequences were as follows:
VecSeqA3 
aaaaacaaacgccccgtaaatcgtgctctcaccgccaagattgatcac-
gaaattacagggcgccgggttccgcgtttcccgatgggaaagcgcggttagttaaactgtgtaccgagagaaatcgtatcac
(SEQ ID NO :10)
VecSeqB3: 
Ccgtacgcgaaatcgttcgtacagtacggtttgtgcggtaatttttccctgaagcgtt-
gaagtacggcgctcatgtgatacgatttctctcggtacacagtttaactaaccgcgctttcccatcgggaaacgcggaacccggcg
ccctgtaatttcgtgatcaatcttggcggtgagagcacgatttacggggcgtttgtttttcagtcgcttacactacacccaacg (SEQ
ID NO:11)
VecSeqA4: 
agataatacaaatgcttaaataaaaaaagacttgatctgattagaccaaatcttttga-
tagtgttatattaataacaaaataaaaaggagtcgctcacgccctgaccaaagtttgtgaacgacatcattcaaagaaaaaaacactg
agttgtttttataatcttgtatatttagatattaaacgatatttaaatatacatcaagata-
tatatttgggtgagcgattccttaaacgaaattgagattaaggagtcgatttttt (SEQ ID NO:12)
VecSeqB4: 
Cgactccttaatctcaatttcgtttaaggaatcgctcacccaaatatatatctt-
gatgtatatttaaatatcgtttaatatctaaatatacaagattataaaaacaactcagtgtttttttctttgaatgatgtcgttcacaaactttg
gtcagggcgtgagcgactcctttttattttgttattaatataacactatcaaaagattt-
ggtctaatcagatcaagtctttttttatttaagcatttgtattatctggtaaacagttaaaactactaaaac (SEQ ID NO:13)
VecSeqA5 : 
cccggcgcttgaggctttctgcctcatgacgtgaaggtggtttgttgccgtgttgtgtgg-
cagaaagaagatagccccgtagtaagttaattttcattaaccaccacgaggcatccctatgtctagtccacatcaggatagcctctta
ccgcgctttgcgcaaggagaagaaggcc (SEQ ID NO:14)
VecSeqB5:

Caacacacaccagacaagggaacttcgtggtagtttcatggccttcttctccttgcgcaaa-
gcgcggtaagaggctatcctgatgtggactagacatagggatgcctcgtggtggttaatgaaaattaacttactacggggctatcttc
tttctgccacacaacacggcaacaaaccaccttcacgtcatgaggcagaaagcctcaa-
gcgccgggcacatcatagcccatatacctgcacgctgaccacactcactttccctgaaaataatccgctcattcagaccgttcacgg
gaaatccgtgtgattgttgccgcatcacgctgcctcccggagtttgtt (SEQ ID NO:15)

### 1. Strain modification

miniVec stable strain 4: A VecSeqA3/ccdB protein expression cassette and a araC/ccdA protein expression cassette were inserted into DH5α strain.

miniVec stable strain 5: A VecSeqA4/ccdB protein expression cassette and two tandem araC/ccdA protein expression cassettes were inserted into DH5a strain.

miniVec stable strain 6: A VecSeqA5/ccdB protein expression cassette and two tandem araC/ccdA protein expression cassettes were inserted into DH5a strain.

### 1.1 Construction of Intermediate Vector (the intermediate vector comprises a homology arm HA that recombines with the bacterial genome):

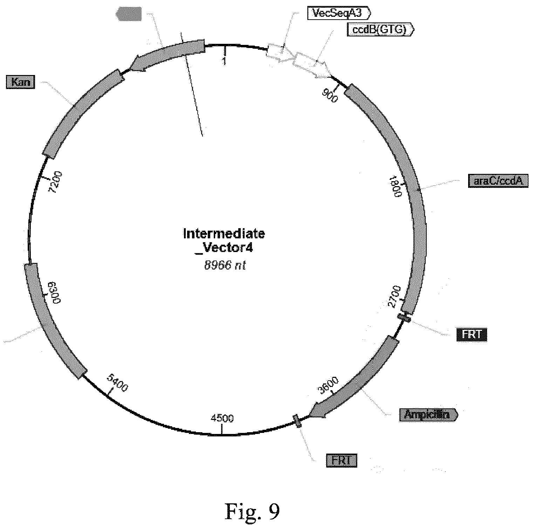

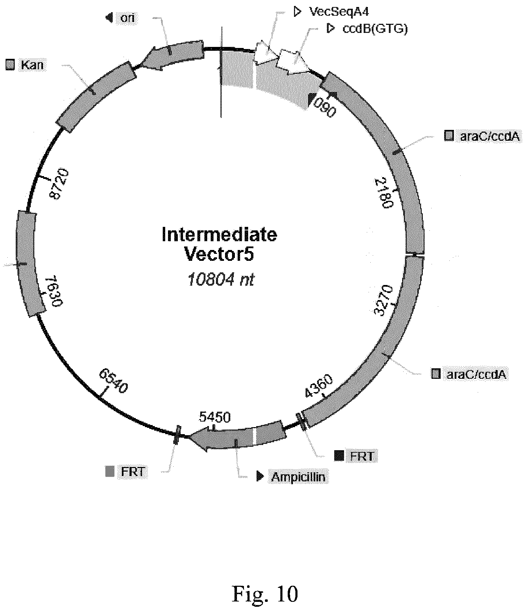

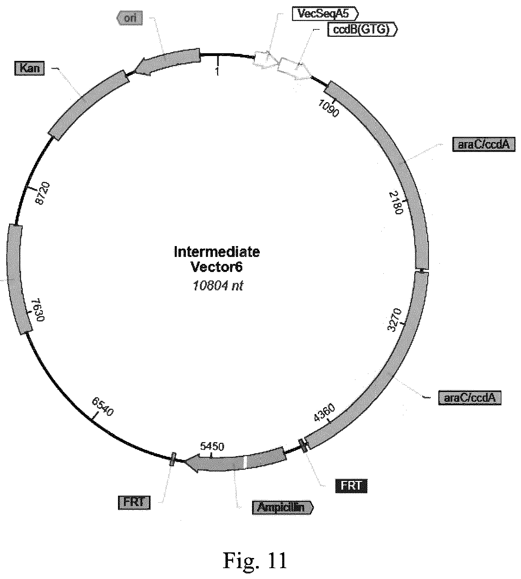

Intermediate Vector 4: expressing a VecSeqA3/ccdB protein and one araC/ccdA expression cassette, as shown in Figure 9;
Intermediate Vector 5: expressing a VecSeqA4/ccdB protein and two araC/ccdA expression cassettes, as shown in Figure 10;
Intermediate Vector 6: expressing a VecSeqA5/ccdB protein and two araC/ccdA expression cassettes, as shown in Figure 11;

### 1.2 Intermediate Vector 4, Intermediate Vector 5 and Intermediate Vector 6 were digested with AarI, and the recovered recombinant fragments were as follows:

Recombinant fragment 4: DH5α HA-VecSeqA3/ccdB-(araC/ccdA)-Frt-Amp-Frt-DH5α HA, 8937 bp;
Recombinant fragment 5: DH5α HA-VecSeqA4/ccdB-2(araC/ccdA)-Frt-Amp-Frt-DH5α HA, 9068 bp;
Recombinant fragment 6: DH5α HA-VecSeqA5/ccdB-2(araC/ccdA)-Frt-Amp-Frt-DH5α HA, 8980 bp;

### 1.3 Construction of Intermediate Strain: It was constructed in 1.4 in Example 1, and was used as the strain for the next experiment.

### 1.4 Preparation of Intermediate Strain electroporation competent cells:

1.5 1 mL of Intermediate Strain bacterial solution was inoculated into 100 mL of Tet LB medium and cultured at 30°C until OD600 was 0.2-0.3. 2 mL of 10% rhamnose was added and the cells were cultured at 37°C for 45 min to induce pHelper Vecto1 to express the recombinase. The Intermediate Strain electroporation competent cells were prepared according to the conventional steps.

1.6 Intermediate Strain competent cells were electroporated with 1 µg of recombinant fragment 4, recombinant fragment 5 and recombinant fragment 6 recovered in 1.2 respectively, coated on Chl+Tet+0.37% arabinose plate, and cultured overnight at 37°C. At this time, the DH5a genome homology arms on the three recombinant fragments were recombined with the DH5a genome under the action of the recombinase, and the recombinant fragments were inserted into the DH5α genome to obtain the initial strains: miniVec stable-Amp strain 4, miniVec stable-Amp strain 5 and miniVec stable-Amp strain 6.

1.7 Colony PCR was performed to identify whether the three miniVec stable strains were inserted with the recombinant target fragments. The positive clones of colony PCR were sent for sequencing to determine the integrity of the recombinant target fragments.

1.8 miniVec stable-Amp electroporation competent cells were prepared according to conventional procedures.

1.9 miniVec stable-Amp strain 4, miniVec stable-Amp strain 5 and miniVec stable-Amp strain 6 competent cells were electroporated with 1µg of pHelper Vector 2 comprising the Flp recombinase (constructed in Example 1), coated on 0.37% arabinose plates, and cultured at 30°C overnight.

1.10 The strains with the Amp resistance gene deleted after FRT recombination were screened, and the positive clones were sent for sequencing to verify the sequence integrity of the inserted target fragments. At this time, the strains were miniVec stable strain 4, miniVec stable strain 5 and miniVec stable strain 6 respectively.

### 2.Construction of miniVec Plasmid

Different miniVec Plasmids in Table 8 were constructed using Gibson homologous recombination reaction:

**Table 8 Summary of different minivec Plasmids**

| Serial number | Vector Name | VecSeqB length |
|---|---|---|
| miniVec Plasmid 8 | pUC-VecSeqB3-EGFP | 228bp |
| miniVec Plasmid 9 | pUC-VecSeqB4-EGFP | 280bp |
| miniVec Plasmid 10 | pUC-VecSeqB5-EGFP | 342bp |

### 3. Test of synergistic effect of VecSeqA/VecSeqB and miniVec stable strains

miniVec stable strain 4 was electroporated with 100 ng of miniVec Plasmid 8, miniVec stable strain 5 was electroporated with miniVec Plasmid 9, and miniVec stable strain 6 was electroporated with miniVec Plasmid 10, and coated on conventional antibiotic free plates and cultured at 37°C overnight. The results are shown in Table 9.

As can be seen from Table 9, the miniVec strains in Group 1*, 3* and 5* did not comprise miniVec Plasmid, and the number of colonies on the antibiotic free plate were more than 2.86E+03 times lower than that on the plate with arabinose, indicating that in the absence of arabinose, araC could inhibit the expression of ccdA, and the ccdB protein killed the strain, and the number of clones was significantly reduced; different VecSeqA would not affect the expression of ccdB. In Group 2*, 4* and 6*, miniVec Plasmids were transferred into miniVec strains, and the positive rate of plasmid screening was more than 59%, indicating that reverse complementation of different VecSeqB and VecSeqA produced steric hindrance effect, which could effectively inhibit toxic gene products, thereby achieving effective plasmid screening effect.

**Table 9 Summary table of data of transformation effect**

| Group | | Number of clones per 1mL transformation bacterial solution | | Positive rate of plasmid screening |
|---|---|---|---|---|
| | | 0.37% arabinose plate | Antibiotic free conventional plate | |
| Group 1* | only minivec strain 4 | 1.63E+09 | 3.30E+05 | 0% |
| Group 2* | minivec Strain 4 + minivec Plasmid 8 | / | 1.08E+07 | 96.9% |
| Group 3* | only minivec strain 5 | 1.43E+09 | 5.00E+05 | 0% |
| Group 4* | minivec Strain 5 + minivec Plasmid 9 | / | 6.70E+06 | 92.5% |
| Group 5* | only minivec strain 6 | 1.12E+09 | 5.01E+04 | 0% |
| Group 6* | minivec Strain 6 + minivec Plasmid 10 | / | 1.22E+05 | 59.0% |

Three clones were picked from Group 2*, Group 4* and Group 6* antibiotic free plates, inoculated into 5 mL LB medium, cultured at 37°C overnight, and miniVec Plasmid was extracted. The yields are summarized in Table 10.

From Table 10, it can be seen that the reverse complementation of different VecSeqB and VecSeqA produced a steric hindrance effect, which inhibited the function of toxic gene products, and thus played a role in plasmid screening. Although the steric hindrance efficiency was different, the toxic protein inhibition efficiency was different, and the plasmid yield was different, plasmid screening and production could all be carried out under the conditions of no antibiotic and no auxiliary inhibitors.

**Table 10 summary of the yields of miniVec Plasmids**

| Group | | Yield (µg/mL) | | |
|---|---|---|---|---|
| | | Clone 1 | Clone 2 | Clone 3 |
| Group 2* | miniVec Strain 4 + miniVec Plasmid 8 | 6.41 | 5.36 | 5.55 |
| Group 4* | miniVec Strain 5 + miniVec Plasmid 9 | 5.84 | 5.71 | 7.10 |
| Group 6* | miniVec Strain 6 + miniVec Plasmid 10 | 9.00 | 8.03 | 10.11 |

### Example 4 Testing various matching systems of toxicity and anti-toxicity

ccdB/ccdA toxic and anti-toxic genes were replaced with Zeta/Epsilon gene and Doc/Phd gene. The UTR of nucleic acid fragment 1 was VecSeqA1. The specific sequences are as follows:
VecSeqA1/Zeta: 
gtttaaggaattttgtggctggccacgccgtaaggtggcaaggaactggttctgatgtggat-
ttacaggagccagaaaagcaaaaaccccgataatcttcttcaactttggcgagtacgaaaagattaccggggcccacttaaaccgt
atagccaacaattcagctatgcggggagtatagttatatgcccggaaaagttcaa-
gacttctttctgtgctcgctccttctgcgcattgtaagtgcaggatggtgtgactgatgcaaatatagtcaattttactgacaaacaattt
gagaatcgcttaaatgataatttagaagaattgattcaaggaaaaaaagcggtt-
gaatcgccaaccgcttttttacttggtgggcaaccagggtcagggaaaaccagtttgcgatcagcaatttttgaagaaacacaaggg
aatgttattgtcattgataatgatacctttaaacaacagcaccctaattttgatgaactag-
tgaaactttatgaaaaagacgtagtaaaacacgttaccccttattctaatcgcatgacagaagcgatcataagccgtttgagcgatca
agggtataatttggtgatcgaaggtacaggacgaacaacagacgttcctattcaaaccg-
caacaatgcttcaagccaaaggttatgaaacaaaaatgtatgtcatggcagtacctaaaatcaactcatatttaggaacaattgaacg
 
atatgaaaccatgtattcagatgatccaatgacagccagggcaacaccaaaacaagcg-
catgatattgttgtcaaaaacttaccgaccaatttagaaacccttcataaaacgggcttatttagcgatataaggctatataacagagaa
ggagtaaaactctattcaagtttggaaacaccttctattagtccaaaagaaacctta-
gaaaaagaattgaatcgtaaagtatcagggaaagaaattcaaccgactttagaacgaatagagcaaaaaatggttctaaataaaca
ccaagagacacctgaatttaaagcaattcaacaaaaattggaaagcttgcagccacctacac-
caccaatacccaaaacacctaaacttccaggtatttaa (SEQ ID NO:16)
 wherein the UTR is positions 1-252, corresponding to positions 1-252 of SEQ ID NO: 1
araBAD-araC/Epsilon: 
gcccgtcacgggcttctcagggcgttttatggcgggtctgc-
tatgtggtgttatctgactttttgctgttcagcagttcctgccctctgattttccagtctgaccacttcggattatcccgtgacaggtcattc
agactggctaatgcacccagtaaggcagcgg-
tatcatcaacaggcttacccgtcttactgtctagactcgaggaattgatatcacccgacaccatcgaatggttaagccactttctctttat
tcaaaacttgttttgagtaacgattcattgaccgccaatactcatgaacggctt-
gtaattcttctaaagaataatcaaaaagatttacacgttttgcaagctttaattggtttaataaattgacttccaataattgagagtcattttt
atttaattcatggttcaaaacatagtttaatactcgattataaacgcttgccga-
taattcgttaatgatctctatttcaaatgttttttcatacgtaactgccatagtgaattcctcctgctagcccaaaaaaacgggtatggaga
aacagtagagagttgcgataaaaagcgtcaggtaggatccgctaatcttatgga-
taaaaatgctatggcatagcaaagtgtgacgccgtgcaaataatcaatgtggacttttctgccgtgattatagacacttttgttacgcgt
ttttgtcatggctttggtcccgctttgttacagaatgcttttaataagcggggttac-
cggtttggttagcgagaagagccagtaaaagacgcagtgacggcaatgtctgatgcaatatggacaattggtttcttctctgaatgg
cgggagtatgaaaagtatggctgaagcgcaaaatgatcccctgctgccggga-
tactcgtttaatgcccatctggtggcgggtttaacgccgattgaggccaacggttatctcgatttttttatcgaccgaccgctgggaat
gaaaggttatattctcaatctcaccattcgcggtcagggggtggtgaaaaatcagggacga-
gaatttgtttgccgaccgggtgatattttgctgttcccgccaggagagattcatcactacggtcgtcatccggaggctcgcgaatggt
atcaccagtgggtttactttcgtccgcgcgcctactgg-
catgaatggcttaactggccgtcaatatttgccaatacggggttctttcgcccggatgaagcgcaccagccgcatttcagcgacctg
tttgggcaaatcattaacgccgggcaaggggaagggcgctattcggagctgctggcga-
taaatctgcttgagcaattgttactgcggcgcatggaagcgattaacgagtcgctccatccaccgatggataatcgggtacgcgag
gcttgtcagtacatcagcgatcacctggcagacagcaattttgatatcgccagcgtcg-
cacagcatgtttgcttgtcgccgtcgcgtctgtcacatcttttccgccagcagttagggattagcgtcttaagctggcgcgaggacca
 
acgtatcagccaggcgaagctgcttttgagcaccacccggatgcctatcgccac-
cgtcggtcgcaatgttggttttgacgatcaactctatttctcgcgggtatttaaaaaatgcaccggggccagcccgagcgagttccgt
gccggttgtgaagaaaaagtgaatgatgtagccgtcaagttgtcataattggtaac-
gaatcagacaattgacggcttgacggagtagcatagggtttgcagaatccctgcttcgtccatttgacaggcacattatgcatcttgg
agtggtgaatccgttagcgaggtgccgccggcttccat (SEQ ID NO:17)
VecSeqA1/Doc: 
gtttaaggaattttgtggctggccacgccgtaaggtggcaaggaactggttctgatgtggat-
ttacaggagccagaaaagcaaaaaccccgataatcttcttcaactttggcgagtacgaaaagattaccggggcccacttaaaccgt
atagccaacaattcagctatgcggggagtatagttatatgcccggaaaagttcaa-
gacttctttctgtgctcgctccttctgcgcattgtaagtgcaggatggtgtgactgataggcatatatcaccggaagaacttattgcgct
tcatgatgcgaatataagccgctacggcggcctgccgggaatgtctgatccgggtaggg-
cagaggccattatcgggagagttcaggccagagttgcctacgaagagatcaccgaccttttcgaagtctccgccacctacctggtg
gctacagcgagagggcatatattcaatgatgccaataagcgtaccgcgctaaacag-
tgcgctgctatttctacgccgtaacggggtgcaggtatttgattcacctgaactggcagaccttactgtaggagctgcgactggcga
gatatctgtatcttctgtcgccgacacgttacgtagattgtatggttctgcggagtag (SEQ ID NO:18)
 wherein the UTR is positions 1-252, corresponding to positions 1-252 of SEQ ID NO: 1
araBAD-araC/Phd:

gcccgtcacgggcttctcagggcgttttatggcgggtctgc-
tatgtggtgttatctgactttttgctgttcagcagttcctgccctctgattttccagtctgaccacttcggattatcccgtgacaggtcattc
agactggctaatgcacccagtaaggcagcgg-
tatcatcaacaggcttacccgtcttactgtctagactcgaggaattgatatcacccgacaccatcgaatggttatcggttaaccagttc
cttgttggtggagtccagggtgtcaaacagggatgcaaattcag-
catccagcgccgcttttttgtaggcttcgaaagtagccttgctgacaattactgctggctcacggcctctgcgggtgatttcaacctctt
ccccggcttcaacattgttgagcacttcagaaaggttgccgcgcgcggtac-
ggaagttaatggattgcatagtgaattcctcctgctagcccaaaaaaacgggtatggagaaacagtagagagttgcgataaaaagc
gtcaggtaggatccgctaatcttatggataaaaatgctatggcatagcaaagtgtgac-
gccgtgcaaataatcaatgtggacttttctgccgtgattatagacacttttgttacgcgtttttgtcatggctttggtcccgctttgttacag
aatgcttttaataagcggggttaccggtttggttagcgagaagagccagtaaaagacgcag-
tgacggcaatgtctgatgcaatatggacaattggtttcttctctgaatggcgggagtatgaaaagtatggctgaagcgcaaaatgatc

ccctgctgccgggatactcgtttaatgcccatctggtggcgggtttaac-
gccgattgaggccaacggttatctcgatttttttatcgaccgaccgctgggaatgaaaggttatattctcaatctcaccattcgcggtca
gggggtggtgaaaaatcagggacgagaatttgtttgccgaccgggtgatatttt-
gctgttcccgccaggagagattcatcactacggtcgtcatccggaggctcgcgaatggtatcaccagtgggtttactttcgtccgcg
cgcctactggcatgaatggcttaactggccgtcaatatttgccaatac-
ggggttctttcgcccggatgaagcgcaccagccgcatttcagcgacctgtttgggcaaatcattaacgccgggcaaggggaagg
gcgctattcggagctgctggcgataaatctgcttgagcaattgttactgcggcgcatggaa-
gcgattaacgagtcgctccatccaccgatggataatcgggtacgcgaggcttgtcagtacatcagcgatcacctggcagacagca
attttgatatcgccagcgtcgcacagcatgtttgctt-
gtcgccgtcgcgtctgtcacatcttttccgccagcagttagggattagcgtcttaagctggcgcgaggaccaacgtatcagccagg
cgaagctgcttttgagcaccacccggatgcctatcgccaccgtcggtcgcaatgttggtttt-
gacgatcaactctatttctcgcgggtatttaaaaaatgcaccggggccagcccgagcgagttccgtgccggttgtgaagaaaaagt
gaatgatgtagccgtcaagttgtcataattggtaacgaatcagacaattgacggcttgac-
ggagtagcatagggtttgcagaatccctgcttcgtccatttgacaggcacattatgcatcttggagtggtgaatccgttagcgaggtg
ccgccggcttccat (SEQ ID NO:19)

### 1. Strain modification

miniVec stable strain 7: a VecSeqA1/zeta protein expression cassette and two tandem araBAD-araC/epsilon protein expression cassettes, and a R6K Rep protein expression cassette were inserted into DH5α strain .

miniVec stable strain 8: a VecSeqA1/ Doc protein expression cassette, two tandem araBAD-araC/Phd protein expression cassettes, and a R6K Rep protein expression cassette were inserted into DH5α strain .

### 1.1 Construction of Intermediate Vector (the intermediate vector comprises a homology arm HA that recombines with the bacterial genome):

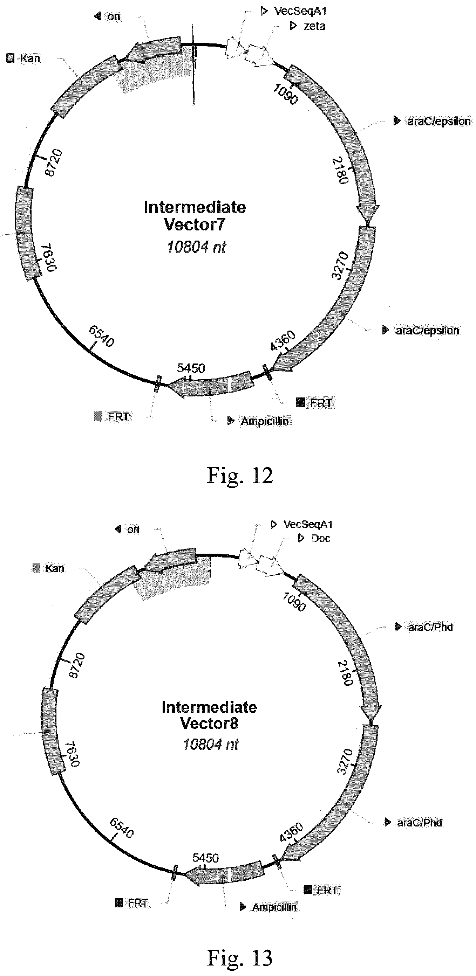

Intermediate Vector7: expressing a VecSeqA1/zeta protein, two araBAD-araC/epsilon proteins, and a R6K Rep protein, as shown in Figure 12;
Intermediate Vector 8: expressing VecSeqA1/Doc protein, two araBAD-araC/Phd proteins, and a R6K Rep protein, as shown in Figure 13;

### 1.2 Intermediate Vector 7 and Intermediate Vector 8 were digested with AarI, and the recovered recombinant fragments were as follows:

Recombinant fragment 7: DH5a HA-VecSeqA1/zeta-2(araC/epsilon)-Frt-Amp-Frt-R6K Rep-DH5a HA, 9922 bp;

Recombinant fragment 8: DH5a HA-VecSeqA1/Doc-2(araC/Phd)-Frt-Amp-Frt-R6K Rep-DH5α HA, 9337 bp.

1.3 Construction of Intermediate Strain: It was constructed in 1.4 in Example 1, and was used as the strain for the next experiment.

1.4 Preparation of Intermediate Strain electroporation competent cells:
1.5 1 mL of Intermediate Strain bacterial solution was inoculated into 100 mL of Tet LB medium and cultured at 30°C until OD600 was 0.2-0.3. 2 mL of 10% rhamnose was added and the cells were cultured at 37°C for 45 min to induce pHelper Vector1 to express the recombinase. The Intermediate Strain electroporation competent cells were prepared according to the conventional steps.

1.6 Intermediate Strain competent cells were electroporated with 1 µg of recombinant fragment 7 and recombinant fragment 8 recovered in 1.2 respectively, coated on Chl+Tet+0.37% arabinose plate, and cultured overnight at 37°C. At this time, the DH5a genome homology arms on the recombinant fragments were recombined with the DH5a genome under the action of the recombinase, and the recombinant fragments were inserted into the DH5α genome to obtain the initial strains: miniVec stable-Amp strain 7 and miniVec stable-Amp strain 8.

1.7 Colony PCR was performed to identify whether the two miniVec stable strains were inserted with the recombinant target fragments. The positive clones of colony PCR were sent for sequencing to determine the integrity of the recombinant target fragments.

1.8 miniVec stable-Amp electroporation competent cells were prepared according to conventional procedures.

1.9 miniVec stable-Amp strain 7 and miniVec stable-Amp strain 8 competent cells were electroporated with 1µg of pHelper Vector 2comprising the Flp recombinase (constructed in Example 1), coated on 0.37% arabinose plates, and cultured at 30°C overnight.

1.10 The strains with the Amp resistance gene deleted after FRT recombination were screened, and the positive clones were sent for sequencing to verify the sequence integrity of the inserted target fragments. At this time, the strains were miniVec stable strain 7 and miniVec stable strain 8 respectively.

### 2.Construction of miniVec Plasmid

Different miniVec Plasmids in Table 11 were constructed using Gibson homologous recombination reaction:

**Table 11 Summary of different minivec Plasmids**

| Serial number | Vector Name | VecSeqB length |
|---|---|---|
| miniVec Plasmid 11 | pR6K-VecSeqB_{1/229}- EGFP - mCherry | 229bp |
| miniVec Plasmid 12 | pUC-VecSeqB_{1/229} -EGFP - mCherry | 229bp |

### 3. Test of the effects of different toxic and anti-toxic systems in miniVec stable strains

miniVec stable strain 7 and miniVec stable strain 8 were electroporated with 100 ng of miniVec Plasmid 11 and miniVec Plasmid 12 respectively, and coated on conventional antibiotic free plates and cultured at 37°C overnight. The results are shown in Table 12.

As can be seen from Table 12, the miniVec strains in Group 1' and 4' did not contain miniVec Plasmid, and the number of colonies on the non-resistant plate was more than 2.99E+03 times lower than that on the plate with arabinose, indicating that in the absence of arabinose, araC could inhibit the expression of different anti-toxic proteins (epsilon/Phd), and different toxic proteins (zeta/Doc) could kill the strains, and the number of colonies was significantly reduced. Therefore, replacing different toxicity/anti-toxic systems was also applicable in the present invention. In Group 2', 3' and Group 5', 6', miniVec Plasmid was transferred into miniVec strain, and the positive rates of plasmid screening ranged from 48.6% to 100%, indicating that the binding of VecSeqB with VecSeqA could effectively inhibit the expression of different toxic proteins (zeta/Doc). In addition, Group 2', 3' and Group 5', 6' were miniVec Plasmids with different Ori, and their screening results were almost the same, indicating that miniVec strains can be used for the preparation of plasmids having various types of Ori such as R6K and pUC.

**Table 12 Summary table of data of transformation effect**

| Group | | 1mL transformation bacterial solution | | Positive rate of plasmid screening |
|---|---|---|---|---|
| | | 0.37% Arabinose plate | Antibiotic free Conventional plate | |
| Group 1' | only minivec strain 7 | 2.00E+07 | 6.68E+03 | 0% |
| Group 2' | miniVec strain 7 + miniVec Plasmid 11 | / | 1.30E+04 | 48.6% |
| Group 3' | minivec strain 7 + minivec Plasmid 12 | / | 4.90E+04 | 86.4% |
| Group 4' | only minivec strain 8 | 1.43E+08 | 4.00E+01 | 0% |
| Group 5' | minivec Strain 8 + minivec Plasmid 11 | / | 5.96E+05 | ≈100% |
| Group 6' | minivec Strain 8 + minivec Plasmid 12 | / | 2.45E+05 | ≈100% |

Three clones were picked from the antibiotic free plates of Group 2', 3', 5' and 6', inoculated into 5 mL of LB medium, cultured overnight at 37°C, and miniVec Plasmid was extracted. The yields are summarized in Table 13. As can be seen from Table 13, although the yields of the plasmids of different groups are different, the overall difference is not large, and resistance-free plasmids can be prepared, indicating that the toxic and anti-toxic gene products of the system of the present invention can be flexibly replaced and are not limited to specific toxic and anti-toxic gene products.

**Table 13 summary of the yields of miniVec Plasmids**

| Group | | Yield (µg/mL) | | |
|---|---|---|---|---|
| | | Clone1 | Clone2 | Clone3 |
| control | Traditional strain DH5a, pRP[Exp]-EGFP-EF1A>mCherry | 3.49 | 3.33 | 2.81 |
| Group 2' | minivec Strain 7 + minivec Plasmid 11 | 2.51 | 5.29 | 4.91 |
| Group 3' | minivec Strain 7 + minivec Plasmid 12 | 4.72 | 4.71 | 4.23 |
| Group 5' | minivec Strain 8 + minivec Plasmid 11 | 7.23 | 5.37 | 4.39 |
| Group 6' | minivec Strain 8 + minivec Plasmid 12 | 4.54 | 4.26 | 4.12 |

It can be seen from all the provided Examples that after the operon inducer is removed, the anti-toxic gene product cannot be expressed normally. However, if the host strain is transformed with the plasmid of the present invention, the small RNA expressed on the plasmid is reversely complementary to the toxic gene product, causing a steric effect, thereby inhibiting the function of the toxic gene product, and thus achieving resistance-free screening of the plasmid without adding antibiotics or auxiliary inhibitors, which can be applied universally.

## Claims

1. Plasmid production system, **characterized in that** it comprises host cell expression cassettes and a plasmid;
wherein the host cell expression cassette comprises a toxic gene expression cassette and an anti-toxic gene expression cassette;
wherein the toxic gene expression cassette comprises a promoter and a nucleic acid fragment 1 for transcribing a toxic gene product;
wherein the anti-toxic gene expression cassette comprises a promoter, an operator and a nucleic acid fragment 2 for transcribing an anti-toxic gene product; wherein the anti-toxic gene product has an inhibition effect on the toxicity produced by the toxic gene product;
and wherein the plasmid comprises a nucleic acid fragment 3 for transcribing a small RNA and an origin of replication, wherein the nucleic acid fragment 3 is reversely complementary to the nucleic acid fragment 1 to produce a steric effect, thereby inhibiting the function of the toxic gene product.

2. Plasmid production system according to claim 1, **characterized in that** the nucleic acid fragment 3 is reversely complementary to the nucleic acid fragment 1 completely, or has at least 20% reverse complementarity or at least 10 continuous bp reverse complementarity.

3. Plasmid production system according to claim 1 or 2, **characterized in that** the nucleic acid fragment 1 comprises a UTR sequence and a toxic gene.

4. Plasmid production system according to any one of claims 1 to 3, **characterized in that** the toxic gene is selected from any one of ccdB, ParE, MazF, Kid, HicA, RelE, VapC, Doc, RatA, HipA, Zeta, ToxN, YeeV, CptA, GhoT, Hok, TisB, SymE and PasA; the nucleic acid fragment 2 is an anti-toxic gene, and the anti-toxic gene is selected from any one of ccdA, ParD, MazE, Kis, HicB, RelB, VapB, Phd, RatB, HipB, Epsilon, ToxI, YeeU, CptB, GhoS, Sok, IstR-1, SymR and PasB/C.

5. Plasmid production system according to any one of claims 1 to 4, **characterized in that** the operon is any one of arabinose operon, Lac operon, rhamnose catabolism operon, tryptophan operon, gab operon and Gal operon.

6. Plasmid production system according to any one of claims 3 to 5, **characterized in that** the small RNA transcribed from the nucleic acid fragment 3 is reversely complementary to the UTR sequence in the nucleic acid fragment 1 and produces a steric effect.

7. Plasmid production system according to any one of claims 3 to 6, **characterized in that** the UTR sequence and the toxic gene are expressed in fusion.

8. Plasmid production system according to any one of claims 1 to 7, **characterized in that** the number of the anti-toxic gene expression cassettes and the number of the toxic gene expression cassettes are both selected from integers ≥1.

9. Plasmid production system according to any one of claims 1 to 8, **characterized in that** the host cell expression cassette further comprises a Rep protein.

10. Plasmid production system according to any one of claims 1 to 9, **characterized in that** the origin of replication is selected from ColE1, pBR322, pMB1, R6K, pUC, F1, p15A, 2 *µ* ori or oriV.

11. Plasmid production system according to any one of claims 1 to 10, **characterized in that** the plasmid further comprises a target gene.

12. Plasmid production system according to any one of claims 1 to 11, **characterized in that** the plasmid comprises a promoter, a target gene, an origin of replication and a nucleic acid fragment 3 for transcribing a small RNA ligated in sequence.

13. Plasmid production system according to any one of claims 1 to 12, further comprising a host bacterium or a host cell, wherein the host bacterium and the host cell are derived from *Escherichia coli, Agrobacterium, Bacillus, Caulobacter* or a yeast.

14. Use of the plasmid production system according to any one of claims 1 to 13 in producing a plasmid or in preparing a product for cell gene therapy.

15. Method for producing a plasmid using the plasmid production system according to any one of claims 1 to 13, **characterized in that** a host integrated with the host cell expression cassette is transferred with the plasmid and is cultured.

## Patentansprüche

1. Plasmidproduktionssystem, **dadurch gekennzeichnet, dass** es Wirtszell-Expressionskassetten und ein Plasmid umfasst;
wobei die Wirtszell-Expressionskassette eine Expressionskassette für ein toxisches Gen und eine Expressionskassette für ein antitoxisches Gen umfasst;
wobei die Expressionskassette für das toxische Gen einen Promotor und ein Nukleinsäurefragment 1 zum Transkribieren eines toxischen Genprodukts umfasst;
wobei die Expressionskassette für das antitoxische Gen einen Promotor, einen Operator und ein Nukleinsäurefragment 2 zum Transkribieren eines antitoxischen Genprodukts umfasst; wobei das antitoxische Genprodukt eine hemmende Wirkung auf die durch das toxische Genprodukt erzeugte Toxizität hat;
und wobei das Plasmid ein Nukleinsäurefragment 3 zum Transkribieren einer kleinen RNA und einen Replikationsursprung umfasst, wobei das Nukleinsäurefragment 3 zu dem Nukleinsäurefragment 1 revers komplementär ist, um einen sterischen Effekt zu erzeugen, wodurch die Funktion des toxischen Genprodukts gehemmt wird.

2. Plasmidproduktionssystem nach Anspruch 1, **dadurch gekennzeichnet, dass** das Nukleinsäurefragment 3 zu dem Nukleinsäurefragment 1 vollständig revers komplementär ist oder mindestens 20 % reverse Komplementarität oder mindestens 10 kontinuierliche bp reverse Komplementarität aufweist.

3. Plasmidproduktionssystem nach Anspruch 1 oder 2, **dadurch gekennzeichnet, dass** das Nukleinsäurefragment 1 eine UTR-Sequenz und ein toxisches Gen umfasst.

4. Plasmidproduktionssystem nach einem der Ansprüche 1 bis 3, **dadurch gekennzeichnet, dass** das toxische Gen aus einem von ccdB, ParE, MazF, Kid, HicA, RelE, VapC, Doc, RatA, HipA, Zeta, ToxN, YeeV, CptA, GhoT, Hok, TisB, SymE und PasA ausgewählt ist; das Nukleinsäurefragment 2 ein antitoxisches Gen ist, und das antitoxische Gen aus einem von ccdA, ParD, MazE, Kis, HicB, RelB, VapB, Phd, RatB, HipB, Epsilon, ToxI, YeeU, CptB, GhoS, Sok, IstR-1, SymR und PasB/C ausgewählt ist.

5. Plasmidproduktionssystem nach einem der Ansprüche 1 bis 4, **dadurch gekennzeichnet, dass** das Operon ein beliebiges aus Arabinose-Operon, Lac-Operon, Rhamnose-Katabolismus-Operon, Tryptophan-Operon, gab-Operon und Gal-Operon ist.

6. Plasmidproduktionssystem nach einem der Ansprüche 3 bis 5, **dadurch gekennzeichnet, dass** die von dem Nukleinsäurefragment 3 transkribierte kleine RNA zu der UTR-Sequenz in dem Nukleinsäurefragment 1 revers komplementär ist und einen sterischen Effekt erzeugt.

7. Plasmidproduktionssystem nach einem der Ansprüche 3 bis 6, **dadurch gekennzeichnet, dass** die UTR-Sequenz und das toxische Gen in Fusion exprimiert werden.

8. Plasmidproduktionssystem nach einem der Ansprüche 1 bis 7, **dadurch gekennzeichnet, dass** die Anzahl der Expressionskassetten für das antitoxische Gen und die Anzahl der Expressionskassetten für das toxische Gen beide aus ganzen Zahlen ≥1 ausgewählt sind.

9. Plasmidproduktionssystem nach einem der Ansprüche 1 bis 8, **dadurch gekennzeichnet, dass** die Wirtszell-Expressionskassette ferner ein Rep-Protein umfasst.

10. Plasmidproduktionssystem nach einem der Ansprüche 1 bis 9, **dadurch gekennzeichnet, dass** der Replikationsursprung aus ColE1, pBR322, pMB1, R6K, pUC, F1, p15A, 2 µ ori oder oriV ausgewählt ist.

11. Plasmidproduktionssystem nach einem der Ansprüche 1 bis 10, **dadurch gekennzeichnet, dass** das Plasmid ferner ein Zielgen umfasst.

12. Plasmidproduktionssystem nach einem der Ansprüche 1 bis 11, **dadurch gekennzeichnet, dass** das Plasmid einen Promotor, ein Zielgen, einen Replikationsursprung und ein Nukleinsäurefragment 3 zum Transkribieren einer kleinen RNA umfasst, die in Sequenz ligiert sind.

13. Plasmidproduktionssystem nach einem der Ansprüche 1 bis 12, ferner umfassend ein Wirtsbakterium oder eine Wirtszelle, wobei das Wirtsbakterium und die Wirtszelle von *Escherichia coli, Agrobacterium, Bacillus, Caulobacter* oder einer Hefe abgeleitet sind.

14. Verwendung des Plasmidproduktionssystems nach einem der Ansprüche 1 bis 13 bei der Herstellung eines Plasmids oder bei der Zubereitung eines Produkts für die Zell-Gentherapie.

15. Verfahren zur Herstellung eines Plasmids unter Verwendung des Plasmidproduktionssystems nach einem der Ansprüche 1 bis 13, **dadurch gekennzeichnet, dass** ein mit der Wirtszell-Expressionskassette integrierter Wirt mit dem Plasmid transformiert und kultiviert wird.

## Revendications

1. Système de production de plasmide, **caractérisé en ce qu'**il comprend des cassettes d'expression de cellule hôte et un plasmide ;
dans lequel la cassette d'expression de cellule hôte comprend une cassette d'expression de gène toxique et une cassette d'expression de gène antitoxique ;
dans lequel la cassette d'expression de gène toxique comprend un promoteur et un fragment d'acide nucléique 1 pour la transcription d'un produit de gène toxique ;
dans lequel la cassette d'expression de gène antitoxique comprend un promoteur, un opérateur et un fragment d'acide nucléique 2 pour la transcription d'un produit de gène antitoxique ; dans lequel le produit de gène antitoxique a un effet d'inhibition sur la toxicité produite par le produit de gène toxique ;
et dans lequel le plasmide comprend un fragment d'acide nucléique 3 pour la transcription d'un petit ARN et une origine de réplication, où le fragment d'acide nucléique 3 est inversement complémentaire au fragment d'acide nucléique 1 pour produire un effet stérique, inhibant ainsi la fonction du produit de gène toxique.

2. Système de production de plasmide selon la revendication 1, **caractérisé en ce que** le fragment d'acide nucléique 3 est complètement inversement complémentaire au fragment d'acide nucléique 1, ou présente une complémentarité inverse d'au moins 20% ou une complémentarité inverse d'au moins 10 pb continues.

3. Système de production de plasmide selon la revendication 1 ou 2, **caractérisé en ce que** le fragment d'acide nucléique 1 comprend une séquence UTR et un gène toxique.

4. Système de production de plasmide selon l'une quelconque des revendications 1 à 3, **caractérisé en ce que** le gène toxique est choisi parmi l'un quelconque de ccdB, ParE, MazF, Kid, HicA, RelE, VapC, Doc, RatA, HipA, Zeta, ToxN, YeeV, CptA, GhoT, Hok, TisB, SymE et PasA ; le fragment d'acide nucléique 2 est un gène antitoxique, et le gène antitoxique est choisi parmi l'un quelconque de ccdA, ParD, MazE, Kis, HicB, ReIB, VapB, Phd, RatB, HipB, Epsilon, Toxl, YeeU, CptB, GhoS, Sok, IstR-1, SymR et PasB/C.

5. Système de production de plasmide selon l'une quelconque des revendications 1 à 4, **caractérisé en ce que** l'opéron est l'un quelconque parmi l'opéron arabinose, l'opéron Lac, l'opéron du catabolisme de rhamnose, l'opéron tryptophane, l'opéron gab et l'opéron Gal.

6. Système de production de plasmide selon l'une quelconque des revendications 3 à 5, **caractérisé en ce que** le petit ARN transcrit à partir du fragment d'acide nucléique 3 est inversement complémentaire à la séquence UTR dans le fragment d'acide nucléique 1 et produit un effet stérique.

7. Système de production de plasmide selon l'une quelconque des revendications 3 à 6, **caractérisé en ce que** la séquence UTR et le gène toxique sont exprimés en fusion.

8. Système de production de plasmide selon l'une quelconque des revendications 1 à 7, **caractérisé en ce que** le nombre de cassettes d'expression de gène antitoxique et le nombre de cassettes d'expression de gène toxique sont tous deux choisis parmi les nombres entiers ≥ 1.

9. Système de production de plasmide selon l'une quelconque des revendications 1 à 8, **caractérisé en ce que** la cassette d'expression de cellule hôte comprend en outre une protéine Rep.

10. Système de production de plasmide selon l'une quelconque des revendications 1 à 9, **caractérisé en ce que** l'origine de réplication est choisie parmi ColE1, pBR322, pMB1, R6K, pUC, F1, p15A, 2 µ ori ou oriV.

11. Système de production de plasmide selon l'une quelconque des revendications 1 à 10, **caractérisé en ce que** le plasmide comprend en outre un gène cible.

12. Système de production de plasmide selon l'une quelconque des revendications 1 à 11, **caractérisé en ce que** le plasmide comprend un promoteur, un gène cible, une origine de réplication et un fragment d'acide nucléique 3 pour la transcription d'un petit ARN ligaturé en séquence.

13. Système de production de plasmide selon l'une quelconque des revendications 1 à 12, comprenant en outre une bactérie hôte ou une cellule hôte, où la bactérie hôte et la cellule hôte sont dérivées *d'Escherichia coli,* d'*Agrobacterium,* de *Bacillus,* de *Caulobacter* ou d'une levure.

14. Utilisation du système de production de plasmide selon l'une quelconque des revendications 1 à 13 dans la production d'un plasmide ou dans la préparation d'un produit pour la thérapie génique cellulaire.

15. Procédé de production d'un plasmide en utilisant le système de production de plasmide selon l'une quelconque des revendications 1 à 13, **caractérisé en ce qu'**un hôte dans lequel est intégrée la cassette d'expression de cellule hôte est transféré avec le plasmide et est mis en culture.
